# EUROPEAN PATENT APPLICATION

(11) **EP 4 501 963 A1**
(43) Date of publication of application: **05.02.2025**
(21) Application number: 23775356.1
(22) Date of filing: 24.03.2023
(51) Int. Cl.: C07K 16/28, A61P 35/00, A61K 39/00

(54) **ANTI-HLA-G CHIMERIC ANTIGEN RECEPTOR, AND USE THEREOF**

(30) Priority: 25.03.2022 KR 20220037684
(71) Applicant: HK inno.N Corporation, Cheongju-si, Chungcheongbuk-do 28158 (KR)
(72) Inventor: KWON, Ki Mun, Seoul 04551 (KR); LEE, Bo Eun, Seoul 04551 (KR); BYUN, Sung Hyun, Seoul 04551 (KR); KIM, Chak Hee, Seoul 04551 (KR); YANG, Yoo Hee, Seoul 04551 (KR); KIM, Ji Eun, Seoul 04551 (KR)
(74) Representative: Maiwald GmbH
(86) International application number: PCT/KR2023/003960
(87) International publication number: WO 2023/182861

(57) **Abstract**

The present invention relates to: a chimeric antigen receptor comprising an antigen-binding site specifically binding to HLA-G, which is an immune checkpoint; immune cells into which the receptor is introduced; and use thereof. The immune cells can effectively remove cancer cells in which an immune checkpoint is expressed, such that even the activation of endogenous immune cells, which had been suppressed by immune checkpoints, can be expected, and thus can be effectively used as an immunotherapeutic method for various cancer diseases related to HLA-G.

## Description

### [Technical Field]

The present invention relates to a chimeric antigen receptor (CAR) including an antigen-binding domain that specifically binds to human leukocyte antigen-G (HLA-G), immune cells into which the receptor has been introduced, and a use thereof.

### [Background Art]

Methods for treating cancer have undergone constant development and change, and methods such as surgery, chemotherapy, and radiotherapy are still used to this day. However, most of these existing cancer treatment methods are effective only in the early stages when cancer has not metastasized, and have the problem that there is a high possibility of recurrence even after surgery when metastasis has already occurred. Therefore, research on methods that utilize immune responses to treat cancer has been ongoing recently.

Among them, there is growing interest in cell therapy methods that use immune cells to enhance the immune response or genetically modify immune cells and then inject them back into the patient. Among the latter methods, chimeric antigen receptor (CAR)-T cells are being studied extensively. CAR-T cells are cells that have been given a targeting function to T cells by introducing artificially designed CAR molecules into T cells, and since CAR molecules show specificity for specific tumor antigens, they may eliminate only tumor cells.

Generally, a CAR includes an extracellular antigen-binding domain, a transmembrane (TM) domain, and an intracellular signaling domain. The extracellular antigen-binding domain may include a single-chain variable fragment (scFv) that targets an identified tumor antigen.

Meanwhile, human major histocompatibility antigen classes I and G, also known as human leukocyte antigen-G (HLA-G), are immune checkpoint factors that inhibit the activation and division of T cells, natural killer (NK) cells, and cytotoxic T cells. Known immune cell receptors for HLA-G include Ig-like transcript 2 (ILT2), ILT4, killer cell immunoglobulin like receptor two Ig domains and long cytoplasmic tail 4 (KIR2DL4), and the like, and all of these receptors have an immunoreceptor tyrosine-based inhibitory (ITIM) motif. When HLA-G binds to the receptor, a signaling pathway that suppresses immune cells is activated by the motif.

Although the expression of HLA-G is limited in normal cells, it has been reported that HLA-G is expressed in many types of cancer cells (Lin A, Yan WH. Human Leukocyte Antigen-G (HLA-G) Expression in Cancers: Roles in Immune Evasion, Metastasis and Target for Therapy. Mol. Med. 21(1), 782-791 (2015)). In addition, it has been reported that the expression level of HLA-G in cancer cells of many cancer patients is correlated with the stage of cancer, and there is also a report that patients expressing HLA-G have a lower survival rate (Ye, Sr., Yang, H., Li, K. et al. Human leukocyte antigen G expression: as a significant prognostic indicator for patients with colorectal cancer. Mod. Pathol. 20, 375-383 (2007)).

### [Disclosure]

### [Technical Problem]

The present inventors have conducted extensive research efforts to develop a chimeric antigen receptor (CAR) capable of effectively treating a disease associated with human leukocyte antigen-G (HLA-G) expression and immune cells including the same. As a result, an anti-HLA-G CAR that specifically binds to HLA-G and T cells and NK cells that express the CAR were produced. In addition, the present inventors confirmed that the T cells and NK cells exhibit high anticancer activity against a cell line expressing HLA-G, thereby completing the present invention.

Therefore, an object of the present invention is to provide an anti-HLA-G CAR and immune cells expressing the receptor.

Another object of the present invention is to provide a nucleic acid including a nucleotide encoding an anti-HLA-G CAR and a vector including the nucleic acid.

Still another object of the present invention is to provide a pharmaceutical composition including the immune cells and a pharmaceutically acceptable carrier.

### [Technical Solution]

To achieve the above-described objects, one aspect of the present invention provides an anti-HLA-G CAR including an antigen-binding domain specifically binding to HLA-G; a TM domain; and an intracellular signaling domain.

In one embodiment of the present invention, the antigen-binding domain specifically binding to HLA-G may include a sequence selected from the following:

(a) a heavy chain variable region including a heavy chain complementarity determining region 1 (CDR1) having an amino acid sequence of SEQ ID NO: 31, a heavy chain CDR2 having an amino acid sequence of SEQ ID NO: 32, and a heavy chain CDR3 having an amino acid sequence of SEQ ID NO: 33; and

a light chain variable region including a light chain CDR1 having an amino acid sequence of SEQ ID NO: 34, a light chain CDR2 having an amino acid sequence of SEQ ID NO: 35, and a light chain CDR3 having an amino acid sequence of SEQ ID NO: 36;

(b) a heavy chain variable region including a heavy chain CDR1 having an amino acid sequence of SEQ ID NO: 37, a heavy chain CDR2 having an amino acid sequence of SEQ ID NO: 38, and a heavy chain CDR3 having an amino acid sequence of SEQ ID NO: 39; and

a light chain variable region including a light chain CDR1 having an amino acid sequence of SEQ ID NO: 40, a light chain CDR2 having an amino acid sequence of SEQ ID NO: 41, and a light chain CDR3 having an amino acid sequence of SEQ ID NO: 42; or

(c) a heavy chain variable region including a heavy chain CDR1 having an amino acid sequence of SEQ ID NO: 43, a heavy chain CDR2 having an amino acid sequence of SEQ ID NO: 44, and a heavy chain CDR3 having an amino acid sequence of SEQ ID NO: 45; and

a light chain variable region including a light chain CDR1 having an amino acid sequence of SEQ ID NO: 46, a light chain CDR2 having an amino acid sequence of SEQ ID NO: 47, and a light chain CDR3 having an amino acid sequence of SEQ ID NO: 48.

As used herein, the term "chimeric antigen receptor (CAR)" refers to a receptor protein formed by combining a receptor site of an immune cell (e.g., a T cell) and a recognition site of a specific antigen expressed on the surface of a cancer cell so that the immune cell may recognize a specific protein. These receptors have a chimeric nature because they combine an antigen-binding site capable of recognizing an antigen expressed by a tumor and a signaling site capable of activating T cells.

In one embodiment of the present invention, the CAR includes an antigen-binding domain, a TM domain, and an intracellular signaling domain. The antigen-binding domain recognizes HLA-G of a target cell and may be an antibody or an antibody fragment. In addition, the antigen-binding fragment may be a single-chain variable fragment (scFv), but is not limited thereto.

In one embodiment of the present invention, the antigen-binding site may include a heavy chain variable region sequence and a light chain variable region sequence, and the two sequences may be connected by a linker including an amino acid sequence of SEQ ID NO: 24. Therefore, the antigen-binding site may be in the form of an scFv.

As used herein, the term "heavy chain" refers to both a full-length heavy chain, which includes a variable region domain VH including a sufficient variable region amino acid sequence to impart specificity to an antigen, and three constant region domains CH1, CH2, and CH3, and a fragment thereof.

As used herein, the term "light chain" refers to both a full-length light chain, which includes a variable region domain VL including a sufficient variable region amino acid sequence to impart specificity to an antigen, and a constant region domain CL, and a fragment thereof.

In one embodiment of the present invention, the antigen-binding domain may include an amino acid sequence of SEQ ID NO: 2, SEQ ID NO: 4, or SEQ ID NO: 6, and specifically, may consist of an amino acid sequence having 80% or more, preferably 90% or more, more preferably 95% or more, and most preferably 99% or more sequence homology to the amino acid sequence of SEQ ID NO: 2, SEQ ID NO: 4 or SEQ ID NO: 6.

In addition, the antigen-binding domain may include:

(a) a heavy chain variable region including an amino acid sequence of SEQ ID NO: 25 or an amino acid sequence having 80% or more sequence homology to SEQ ID NO: 25 and a light chain variable region including an amino acid sequence of SEQ ID NO: 26 or an amino acid sequence having 80% or more sequence homology to SEQ ID NO: 26;

(b) a heavy chain variable region including an amino acid sequence of SEQ ID NO: 27 or an amino acid sequence having 80% or more sequence homology to SEQ ID NO: 27 and a light chain variable region including an amino acid sequence of SEQ ID NO: 28 or an amino acid sequence having 80% or more sequence homology to SEQ ID NO: 28; or

(c) a heavy chain variable region including an amino acid sequence of SEQ ID NO: 29 or an amino acid sequence having 80% or more sequence homology to SEQ ID NO: 29 and a light chain variable region including an amino acid sequence of SEQ ID NO: 30 or an amino acid sequence having 80% or more sequence homology to SEQ ID NO: 30.

The CAR may further include a leader sequence (LS). The LS refers to a sequence that allows a CAR to be translocated to the cell membrane to be expressed and exposed on the cell surface.

In one embodiment of the present invention, the LS may be an LS derived from cluster of differentiation 8 alpha (CD8α), a human immunoglobulin heavy chain, or a human granulocyte-macrophage colony-stimulating factor (hGM-CSF) receptor α-chain, and specifically, may be a CD8α-derived LS.

The CD8α-derived LS may include an amino acid sequence of SEQ ID NO: 8, and specifically, may consist of an amino acid sequence having 80% or more, preferably 90% or more, more preferably 95% or more, and most preferably 99% or more sequence homology to the amino acid sequence of SEQ ID NO: 8.

In the CAR of the present invention, the antigen-binding domain specifically binding to HLA-G may be connected to the TM domain by a hinge. The hinge may be derived from the group consisting of a hinge of CD8α, a hinge of IgG1, a hinge of IgG4, a hinge of IgD, IgG1 CH3, an extracellular domain of CD28, or a combination thereof, and specifically, may be derived from CD8α. The hinge derived from CD8α may include an amino acid sequence of SEQ ID NO: 10, and specifically, may consist of an amino acid sequence having 80% or more, preferably 90% or more, more preferably 95% or more, and most preferably 99% or more sequence homology to the amino acid sequence of SEQ ID NO: 10.

In the CAR of the present invention, the TM domain is a domain that connects the antigen-binding domain and the intracellular signaling domain via the cell membrane. The TM domain may be a TM domain of a protein selected from the group consisting of CD8α, T cell receptors, CD28, CD3 epsilon (CD3ε), CD45, CD4, CD5, CD9, CD16, CD22, CD33, CD37, CD64, CD80, CD86, CD99, CD134, CD137, and alpha (α), beta (β), or zeta (ζ) of CD154, and specifically, may be a TM domain of CD8α.

The TM domain of CD8α may include an amino acid sequence of SEQ ID NO: 12, and specifically, may consist of an amino acid sequence having 80% or more, preferably 90% or more, more preferably 95% or more, and most preferably 99% or more sequence homology to the amino acid sequence of SEQ ID NO: 12.

In the CAR of the present invention, the intracellular signaling domain (ICD) is a site that generates a signal that activates an immune response of immune cells when the antigen-binding site of the extracellular domain binds to HLA-G.

The intracellular signaling domain may be derived from a protein selected from the group consisting of: CD3ζ, Fcy receptor (FcyR), inducible T-cell costimulatory (ICOS; CD278), 4-1BB (CD137, tumor necrosis factor receptor superfamily member 9 (TNFRSF9)), OX40 (CD134), CD27, CD28, interleukin-2 receptor beta (IL-2Rβ), IL-15R-α, CD40, myeloid differentiation primary response 88 (MyD88), DNAX-activating protein 10 (DAP10), DAP12, major histocompatibility complex (MHC) class I molecules, tumor necrosis factor (TNF) receptor proteins, immunoglobulin-like proteins, cytokine receptors, integrins, signaling lymphocytic activation molecule (SLAM) protein, activating NK cell receptors, B and T lymphocyte attenuator (BTLA), toll-like receptors (TLRs), CD2, CD7, CD30, CD99, CDS, intercellular adhesion molecule-1 (ICAM-1), B7-H3 (CD276), glucocorticoid-induced tumor necrosis factor receptor (TNFR)-related protein (GITR), B-cell activating factor (BAFF) receptor (BAFFR), lymphotoxin-β receptor interacting cytokine (LIGHT), herpesvirus entry mediator (HVEM) (LIGHT receptor (LIGHTR)), killer cell immunoglobulin like receptor, two Ig domains and short cytoplasmic tail 2 (KIRDS2), signaling lymphocytic activation molecule (SLAM) family member 7 (SLAMF7), NKp80 (killer cell lectin-like receptor subfamily F, member 1 (KLRF1)), NKp44, NKp30, NKp46, CD19, CD4, CD8α, CD8β, IL2Ry, IL7Rα, integrin subunit alpha 4 (ITGA4), very late antigen-1 (VLA1), CD49a, IA4, CD49D, integrin subunit alpha 6 (ITGA6), VLA-6, CD49f, integrin subunit alpha D (ITGAD), CD11d, integrin subunit alpha E (ITGAE), CD103, integrin subunit alpha L (ITGAL), CD11a, lymphocyte function-associated antigen-1 (LFA-1; CD11a/CD18), integrin subunit alpha M (ITGAM), CD11b, integrin subunit alpha X (ITGAX), CD11c, integrin beta-1 (ITGB1), CD29, integrin beta-2 (ITGB2), CD18, integrin beta-7 (ITGB7), natural killer group 2 member D (NKG2D; killer cell lectin like receptor K1 (KLRK1)), natural killer group 2 member C (NKG2C; (killer cell lectin like receptor C2 (KLRC2)), tumor necrosis factor receptor 2 (TNFR2), TNF-related activation-induced cytokine (TRANCE; receptor activator of nuclear factors κB ligand (RANKL)), DNAX accessory molecule-1 (DNAM1; CD226), SLAMF4 (CD244, 2B4), CD84, CD96 (tactile), carcinoembryonic antigen-related cell adhesion molecule 1 (CEACAM1), cytotoxic and regulatory T cell molecule (CRTAM), Ly9 (CD229), CD160 (BY55), P-selectin glycoprotein ligand 1 (PSGL1; CD162), CD100 (Semaphorin 4D (SEMA4D)), CD69, SLAMF6 (NK-T-B-antigen (NTB-A)), Ly108, SLAMF1 (CD150, IPO-3), B lymphocyte activation modulator and enhancer (BLAME; SLAMF8), lymphotoxin-beta receptor (LTBR), linker for activation of T cells (LAT), glutamate decarboxylase (GADS), Src sequence homology 2 (SH2) domain-containing leukocyte phosphoprotein of 76-kDa (SLP-76), phosphoprotein associated with glycosphingolipid-enriched microdomain/C-terminal Src kinase (Csk)-binding protein (PAG/Cbp), CD19a, and CD83-specific ligands, and specifically, may be a CD3ζ-derivd intracellular signaling domain.

The CD3ζ-derivd intracellular signaling domain may include an amino acid sequence of SEQ ID NO: 16, and specifically, may consist of an amino acid sequence having 80% or more, preferably 90% or more, more preferably 95% or more, and most preferably 99% or more sequence homology to the amino acid sequence of SEQ ID NO: 16.

In addition, the intracellular signaling domain may further include a costimulatory domain to further activate immune cells and increase their viability. The costimulatory domain may be derived from a protein selected from the group consisting of OX40, CD2, CD27, CD28, CD99, ICAM-1, LFA-1 (CD11a/CD18), ICOS (CD278), and 4-1BB (CD137), and specifically, may be derived from 4-1BB.

The 4-1BB-derived costimulatory domain may include an amino acid sequence of SEQ ID NO: 14, and specifically, may consist of an amino acid sequence having 80% or more, preferably 90% or more, more preferably 95% or more, and most preferably 99% or more sequence homology to the amino acid sequence of SEQ ID NO: 14.

In one embodiment of the present invention, the CAR may include an amino acid sequence of SEQ ID NO: 18, SEQ ID NO: 20, or SEQ ID NO: 22, and specifically, may consist of an amino acid sequence having 80% or more, preferably 90% or more, more preferably 95% or more, and most preferably 99% or more sequence homology to the amino acid sequence of SEQ ID NO: 18, SEQ ID NO: 20, or SEQ ID NO: 22.

Another aspect of the present invention is an isolated nucleic acid including a nucleotide sequence encoding the anti-HLA-G CAR.

As used herein, the term "nucleic acid" has a comprehensive meaning including DNA and RNA molecules, and nucleotides, which are the basic units of nucleic acid molecules, include not only natural nucleotides but also analogs in which the sugar or base moieties are modified (Scheit, Nucleotide Analogs, John Wiley, New York (1980); Uhlman and Peyman, Chemical Reviews (1990) 90:543-584). The sequence of the nucleic acid molecule encoding the anti-HLA-G CAR of the present invention may be modified, and the modifications include addition, deletion, or non-conservative or conservative substitution of nucleotides.

In one embodiment of the present invention, the isolated nucleic acid including a nucleotide sequence encoding the anti-HLA-G CAR includes a nucleotide sequence encoding an antigen-binding site that specifically binds to HLA-G. The nucleotide sequence encoding the antigen-binding site may include a nucleotide sequence of SEQ ID NO: 1, SEQ ID NO: 3, or SEQ ID NO: 5, and specifically, may consist of a nucleotide sequence having 80% or more, preferably 90% or more, more preferably 95% or more, and most preferably 99% or more sequence homology to the nucleotide sequence of SEQ ID NO: 1, SEQ ID NO: 3, or SEQ ID NO: 5.

In one embodiment of the present invention, the isolated nucleic acid including a nucleotide sequence encoding the anti-HLA-G CAR may include a nucleotide sequence of SEQ ID NO: 17, SEQ ID NO: 19, or SEQ ID NO: 21, and specifically, may consist of a nucleotide sequence having 80% or more, preferably 90% or more, more preferably 95% or more, and most preferably 99% or more sequence homology to the nucleotide sequence of SEQ ID NO: 17, SEQ ID NO: 19, or SEQ ID NO: 21.

Still another aspect of the present invention is a recombinant vector including the nucleic acid molecule.

The vector used in the present invention may be a variety of vectors known in the art, and depending on the type of host cells in which the antigen receptor is to be produced, expression regulatory sequences such as promoters, terminators, enhancers, sequences for membrane targeting or secretion, and the like may be appropriately selected and combined in various ways according to the purpose.

Methods for transducing the vector of the present invention into cells and expressing the same are well known in the art. The vector may be easily introduced into a host cell, such as a mammalian, bacterial, yeast, or insect cell, by methods known in the art. For example, the vector can be delivered into a host cell by physical, chemical, or biological means. The physical means include calcium phosphate precipitation, lipofection, particle bombardment, microinjection, electroporation, and the like. The chemical means include colloidal dispersion systems, for example, macromolecular complexes, nanocapsules, microspheres, beads, and lipid-based systems including oil-in-water emulsions, micelles, mixed micelles, and liposomes. In addition, the biological means include the use of above-described DNA or RNA vectors, such as the lentiviruses and retroviruses.

The vector of the present invention includes a plasmid vector, a cosmid vector, a bacteriophage vector, and a viral vector, but is not limited thereto. In addition to expression regulatory factors such as promoters, operators, initiation codons, termination codons, polyadenylation signals, and enhancers, suitable vectors may include a signal sequence or a leader sequence for membrane targeting or secretion and may be produced in various ways depending on the purpose.

In one embodiment of the present invention, a lentiviral vector was used. In one embodiment of the present invention, the vector further includes a promoter. The promoter may be, for example, an elongation factor 1 (EF-1) promoter, but is not limited thereto.

The present invention also provides an immune cell expressing the above-described anti-HLA-G CAR on the cell surface.

In the present invention, the immune cell may be selected from the group consisting of natural killer cells, T cells, natural killer T cells, B cells, macrophages, dendritic cells, natural killer dendritic cells, mast cells, and precursor cells thereof.

The anti-HLA-G CAR may be introduced into immune cells via the above-described vector, and a standard technique suitable for the host cell may be selected as an introduction method, as known in the art.

In one embodiment of the present invention, T cells and NK cells expressing the CAR were produced by transfecting T cells and NK cells with a recombinant vector including a polynucleotide capable of encoding the anti-HLA-G CAR. Specifically, the anti-HLA-G CAR encoding sequence included in the recombinant vector is transcribed into mRNA, and an anti-HLA-G CAR polypeptide is translated from the mRNA and displayed on the surface of the T cells and NK cells. When necessary, the T cells and NK cells transfected with the recombinant vector may be cultured.

Immune cells expressing the anti-HLA-G CAR may recognize HLA-G as an antigen and specifically bind to HLA-G, and after binding, it may induce immune cell activation through the intracellular signaling domain and induce tumor-specific apoptosis.

Therefore, yet another aspect of the present invention provides a pharmaceutical composition for use in the prevention or treatment of cancer, including immune cells and a pharmaceutically acceptable carrier.

In the present invention, the pharmaceutical composition may be used for the treatment of cancer that overexpresses HLA-G, but is not limited thereto. The cancer may be selected from the group consisting of pancreatic cancer, breast cancer, ovarian cancer, glioma, cervical cancer, endometrial cancer, esophageal cancer, stomach cancer, liver cancer, lung cancer, colon cancer, nasopharyngeal cancer, oral cancer, thyroid cancer, prostate cancer, renal cancer, gallbladder cancer, bile duct cancer, blood cancer, melanoma, and the like.

The blood cancer may be acute myeloid leukemia (AML), acute lymphoblastic leukemia (ALL), chronic myelogenous leukemia (CML), multiple myeloma (MM), or lymphoma.

As used herein, the term "prevention" means any act of suppressing or delaying the progression of a disease by administering the composition of the present invention, and "treatment" means suppressing, alleviating, or eliminating the development of a disease.

The pharmaceutical composition of the present invention may further include a pharmaceutically acceptable carrier, and the pharmaceutically acceptable carrier is one that is commonly used in the preparation of formulations, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil, but is not limited thereto. In addition to the above ingredients, the composition for preventing or treating cancer of the present invention may further include a lubricant, a wetting agent, a sweetening agent, a flavoring agent, an emulsifier, a suspending agent, a preservative, and the like. Suitable pharmaceutically acceptable carriers and formulations are described in detail in the document [Remington's Pharmaceutical Sciences (19th ed., 1995)].

In addition, the pharmaceutical composition of the present invention may be administered by oral administration, infusion, intravenous injection, intramuscular injection, subcutaneous injection, intraperitoneal injection, intrarectal administration, topical administration, intranasal injection, and the like, but the administration method is not limited thereto. In the case of oral administration, since proteins or peptides are digested, the oral composition may be formulated to coat the active agent or protect it from decomposition in the stomach, and the composition of the present invention may be administered by any device that allows the active substance to move to the target cell.

The suitable dosage of the pharmaceutical composition of the present invention varies depending on factors such as the formulation method, administration method, the patient's age, weight, sex, and pathological conditions, food, administration time, administration route, excretion rate, and response sensitivity, and a physician of ordinary skill may easily determine and prescribe a dosage effective for the desired treatment or prevention. The composition may preferably include immune cells expressing the CAR according to the present invention in an amount of 1 to 10 times the number of tumor cells (e.g., pancreatic cancer, breast cancer, ovarian cancer or glioma) in the subject to be treated, but is not limited thereto.

According to one embodiment of the present invention, the pharmaceutical composition of the present invention may include, in a single dose, immune cells (e.g., T cells or natural killer cells) in an amount of 1 to 10 times the number of tumor cells, e.g., pancreatic cancer cells, breast cancer cells, ovarian cancer cells, or glioma cells, in the subject to be treated.

As used herein, the term "pharmaceutically effective amount" refers to an amount sufficient for the treatment, prevention, and diagnosis of a disease.

The pharmaceutical composition of the present invention may be prepared in a unit dosage form or it may be prepared by placing it into a multi-dose container by formulating it using a pharmaceutically acceptable carrier and/or excipient according to a method that may be easily implemented by a person skilled in the art to which the present invention pertains. At this time, the formulation may be in the form of a solution, suspension or emulsion in an oil or an aqueous medium, or it may be in the form of an extract, powder, suppository, granules, tablets, or capsules, and may further contain a dispersant or stabilizer.

The composition of the present invention may be administered as an individual therapeutic agent or in combination with other therapeutic agents, and may be administered sequentially or simultaneously with conventional therapeutic agents.

Yet another aspect of the present invention is a method of diagnosing, preventing, or treating a disease associated with HLA-G overexpression, for example, cancer, the method including administering the anti-HLA-G CAR, or an immune cell expressing the CAR, or the pharmaceutical composition of the present invention in a pharmaceutically effective amount to a subject, for example, a human or a non-human mammal. The present invention also provides a method of diagnosing a disease associated with HLA-G overexpression, for example, cancer, the method including administering the anti-HLA-G CAR or an immune cell expressing the CAR of the present invention to an individual, for example, a human or a non-human mammal, in need thereof.

As used herein, the term "individual" refers to any animal, including a human, a monkey, a cow, a horse, a sheep, a pig, a chicken, a turkey, a quail, a cat, a dog, a mouse, a rat, a rabbit, or a guinea pig, which has developed or may develop a disease associated with HLA-G overexpression.

### [Advantageous Effects]

Since the CAR according to the present invention has excellent specificity and affinity for HLA-G, immune cells expressing the anti-HLA-G CAR have excellent cytotoxicity against cancer cells and can be used for the treatment of various cancers associated with the overexpression of HLA-G.

### [Description of Drawings]

FIGS. 1A to 1C are diagrams illustrating the structures of the HLA-G CAR (antibody fragment 1 CAR), HLA-G CAR (antibody fragment 2 CAR), and HLA-G CAR (antibody fragment 3 CAR) constructs of the present invention.

FIGS. 2A to 2C are diagrams illustrating the structures of the pLV-EF1A-HLA-G CAR (antibody fragment 1 CAR), pLV-EF1A-HLA-G CAR (antibody fragment 2 CAR), and pLV-EF1A-HLA-G CAR (antibody fragment 3 CAR) plasmids of the present invention.

FIG. 3 is a diagram illustrating the structure of the pLV-EF1A-B2MIRES-HLA-G-CMV-EGFP-T2A-PuroR plasmid of the present invention.

FIG. 4 is a diagram confirming the HLA-G expression rate in the SK-OV-3-HLA-G- and MDA-MB-231-HLA-G-overexpressing cell lines of the present invention.

FIGS. 5A to 5C are diagrams confirming the expression rate of the CAR on the surface of the anti-HLA-G CAR-expressing T cells of the present invention.

FIGS. 6A to 6C are diagrams illustrating the anticancer efficacy of the anti-HLA-G CAR-expressing T cells of the present invention against SK-OV-3-HLA-G, MDA-MB-231-HLA-G, SK-OV-3, and MDA-MB-231 cell lines.

FIG. 7 is a diagram illustrating the activity of the anti-HLA-G CAR-expressing T cells of the present invention against SK-OV-3-HLA-G, MDA-MB-231-HLA-G, SK-OV-3, and MDA-MB-231 cell lines, as confirmed by the secretion of interferon gamma.

FIG. 8 is a diagram illustrating the expression rate of the CAR on the surface of the anti-HLA-G CAR-expressing T cells cultured with IL-2 and IL-7/IL-15 cytokines in the present invention.

FIG. 9 is a diagram illustrating the anticancer efficacy of the anti-HLA-G CAR-expressing T cells cultured with IL-2 and IL-7/IL-15 cytokines in the present invention against SK-OV-3-HLA-G and SK-OV-3 cell lines.

FIG. 10 is a diagram illustrating the activity of the anti-HLA-G CAR-expressing T cells cultured with IL-2 and IL-7/IL-15 cytokines in the present invention against SK-OV-3-HLA-G and SK-OV-3 cell lines, as confirmed by the secretion of interferon gamma.

FIG. 11 is a diagram illustrating the expression rate of CD3 and CD56 in the anti-HLA-G CAR-expressing NK cells of the present invention.

FIG. 12 is a diagram illustrating the expression rate of the CAR on the surface of the anti-HLA-G CAR-expressing NK cells of the present invention.

FIG. 13 is a diagram illustrating the anticancer efficacy of the anti-HLA-G CAR-expressing NK cells of the present invention against SK-OV-3-HLA-G and SK-OV-3 cell lines.

### [Modes of the Invention]

Hereinafter, the present invention will be described in more detail through examples. These examples are only intended to illustrate the present invention, and it will be obvious to those skilled in the art that the scope of the present invention is not to be construed as being limited by these examples.

### Example 1: Cell lines and culture

Human ovarian cancer cell line SK-OV-3 and human triple-negative breast cancer cell line MDA-MB-231 were supplied by the Korea Cell Line Bank (Korea). The SK-OV-3 and MDA-MB-231 were cultured in a Roswell Park Memorial Institute (RPMI 1640; Gibco, USA) medium containing 10% fetal bovine serum (FBS) (Gibco, USA) and 1% antibiotic-antimycotic (Gibco, USA).

### Example 2: Production and culture of HLA-G-overexpressing cell lines

### 2-1. Production of B2M-HLA-G-EGFP-PuroR gene expressing lentivirus

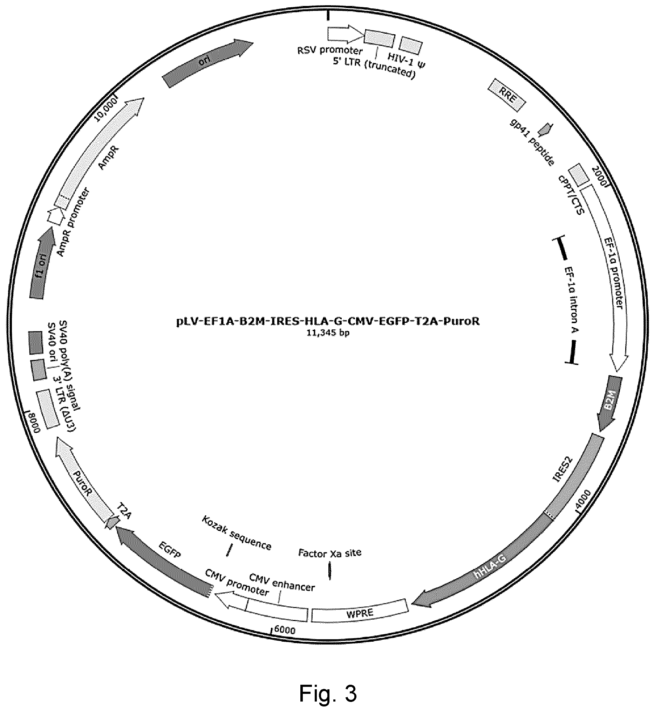

A lentivirus for delivery of B2M-HLA-G-EGFP-PuroR gene was produced using plasmid DNA transfection. The TransIT-293 transfection reagent (Mirus Bio LLC, USA) was used, and transfection was performed according to the manufacturer's protocol. 5×10⁶ cells of a Lenti-X^{™} 293T cell line (Clontech, USA) were seeded in a 100-mm dish the day before the experiment, and on the following day, the cells were transfected with a pLV-EF1A-B2M-IRES-HLA-G-CMV-EGFP-T2A-PuroR lentivirus vector (FIG. 3), a gag-pol expression vector, and a vesicular stomatitis virus-G (VSV-G) envelope expression vector. After culturing the cells for approximately 72 hours, all cell supernatants were harvested. The supernatant was filtered through a 0.45 µm filter (Millipore, USA) to remove cell debris, and the filtrate containing the B2M-HLA-G-EGFP-PuroR lentivirus was stored frozen at -80 °C until use.

### 2-2. Production and culture of SK-OV-3 and MDA-MB-231 cell lines expressing B2M-HLA-G-EGFP-PuroR gene

The day before the experiment, 1×10⁶ cells of each of SK-OV-3 and MDA-MB-231 cell lines were seeded in 100 mm dishes, and 3 mL of the previously produced B2M-HLA-G-EGFP-PuroR lentivirus was mixed with 7 mL of fresh medium per dish and added to the dishes. Additionally, polybrene was added to the medium at a concentration of 8 µg/mL. Then, after 24 hours, the medium was replaced with fresh medium containing 2 µg/mL puromycin (Sigma-Aldrich, USA). When selection by puromycin had progressed to a certain extent, the cells were subcultured at a concentration of 0.5 cells/well in 96-well plates to separate HLA-G-overexpressing cells into single cells. After the final selection was completed, puromycin was added to the medium at a concentration of 0.5 µg/mL. Several wells with high enhanced green fluorescent protein (EGFP) fluorescence signals were selected by observing under a fluorescence microscope, and when the wells were full of cells, the cells were harvested and cultured sequentially in 48-well, 24-well, 12-well, and 6-well plates, and T25 and T75 flasks. Finally, the cells were cultured in a T175 flask to produce a cell stock.

### 2-3. Confirmation of HLA-G expression in SK-OV-3 and MDA-MB-231 cell lines expressing B2M-HLA-G-EGFP-PuroR gene

The expression of HLA-G in the HLA-G-overexpressing cell lines produced in 2-2 above was confirmed as described below.

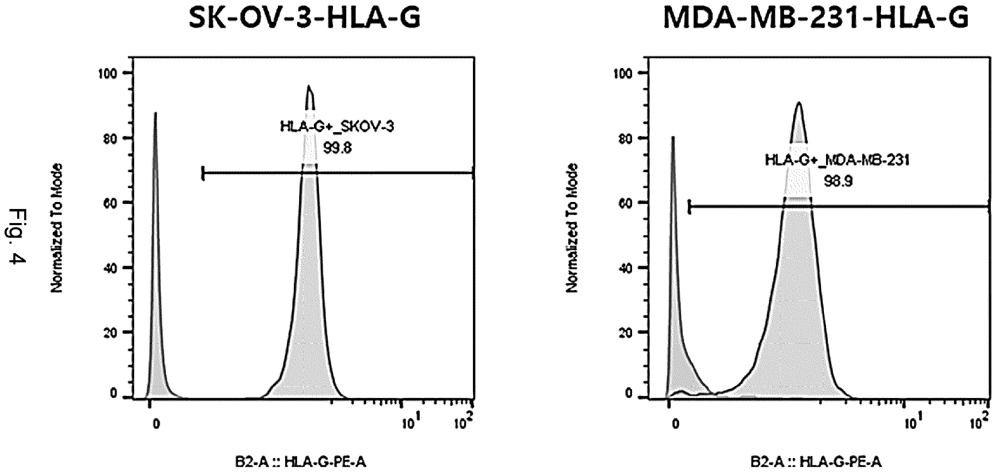

1×10⁵ cells of the B2M-HLA-G-EGFP-PuroR gene expressing SK-OV-3 and MDA-MB-231 cell lines were suspended in 100 µL of Dulbecco's phosphate-buffered saline (D-PBS) (Gibco, USA) to prepare cell samples. 1 µg of the anti-HLA-G (TANKIO17) antibody was added to the cell samples, and the cells were allowed to react at 4 °C for 30 minutes. After the reaction was completed, the cells were washed twice with D-PBS and resuspended. 0.25 µg of R-phycoerythrin AffiniPure F(ab')₂ fragment goat anti-human IgG (H+L) (Jackson ImmunoResearch, USA) was added to the cell samples, and the cells were allowed to react at 4 °C for 30 minutes. After the reaction, the cells were washed twice with D-PBS, and the expression of HLA-G was confirmed by flow cytometry. As a result, it was confirmed that approximately 99.8% of HLA-G was expressed on the surface of the SK-OV-3 cells to which the B2M-HLA-G-EGFP-PuroR lentivirus was delivered, and that approximately 98.9% of HLA-G was expressed on the surface of the MDA-MB-231 cells (FIG. 4).

### Example 3: Construction of CAR

The CAR of the present invention was artificially synthesized.

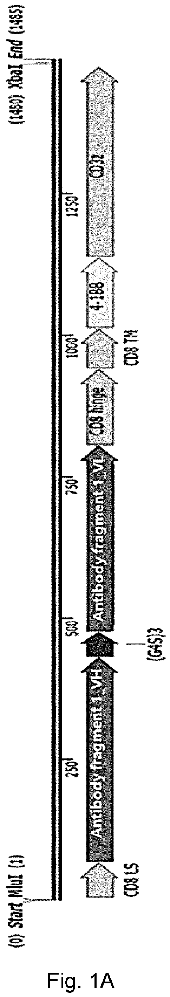

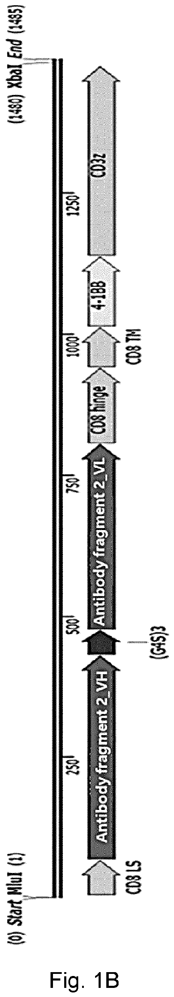

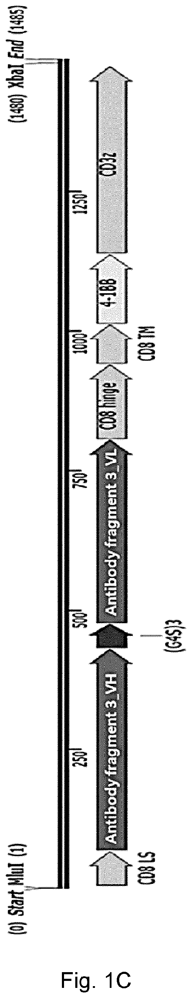

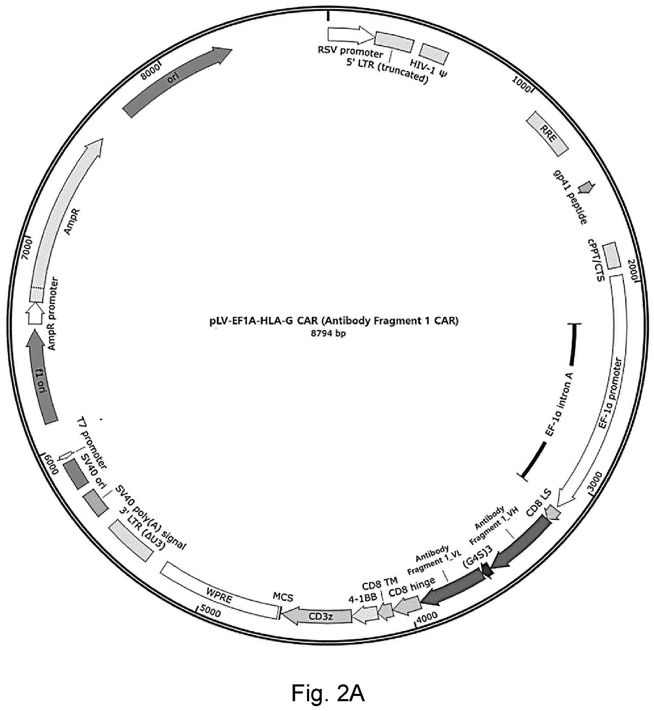

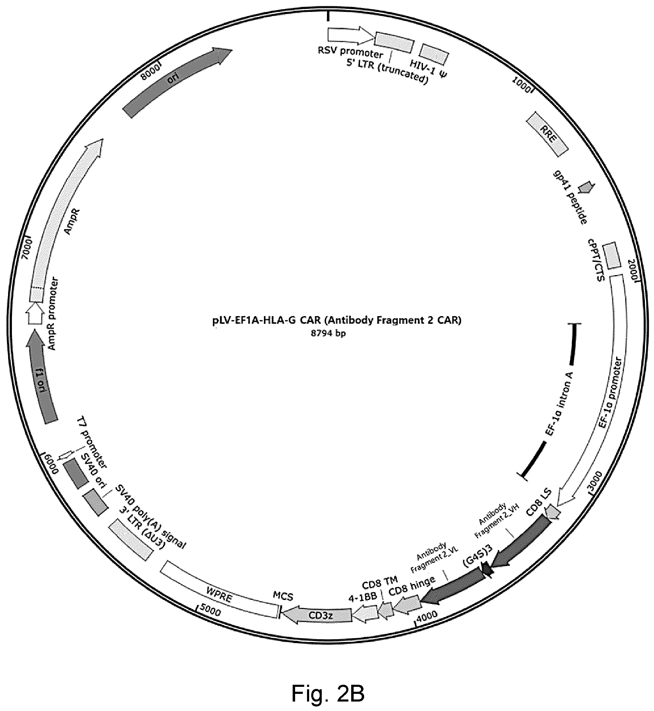

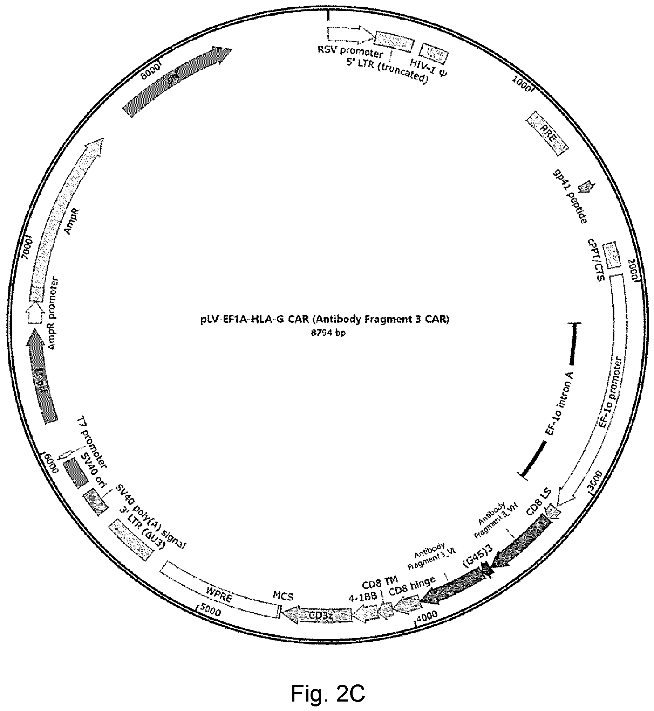

More specifically, the following extracellular domain and intracellular domain coding sequences were artificially synthesized through splicing by overlap extension polymerase chain reaction (SOE-PCR) to produce CARs (FIGS. 1A to 1C). The PCR product was digested with MluI and XbaI and then inserted into the MluI and XbaI sites of the pLV-EF1A-MCS vector, which is a third-generation self-inactivating lentiviral expression vector (FIGS. 2A to 2C).

- Extracellular domains: CD8α-derived LS, HLA-G binding domain (antibody fragment 1, antibody fragment 2, or antibody fragment 3; scFv), CD8α-derived hinge, and CD8α-derived TM domain

- - Intracellular domain: intracellular signaling domain of 4-1BB and CD3ζ

CARs according to an example of the present invention are summarized in Table 1 below. The domains of the CAR are connected in tandem with each other and also in frame. Specifically, a CD8α-derived LS, an HLA-G binding domain (in the form of scFv), a CD8α-derived hinge, a CD8α-derived TM domain, a 4-1BB-derived intracellular signaling domain, a CD3ζ-derived intracellular signaling domain, and a stop codon TAA are connected.

**[Table 1]**

| Serial NO. | Abbreviation | LS | scFv | Hinge | TM | Signaling domain-1 | Signaling domain-2 |
|---|---|---|---|---|---|---|---|
| S1 | CAR 1 | CD8α | Antibody fragment 1 | CD8α | CD8α | 4-1BB | CD3ζ |
| S2 | CAR 2 | CD8α | Antibody fragment 2 | CD8α | CD8α | 4-1BB | CD3ζ |
| S3 | CAR 3 | CD8α | Antibody fragment 3 | CD8α | CD8α | 4-1BB | CD3ζ |

The sequence information of the CARs according to an example of the present invention and the domains used in their production are summarized in Tables 2 and 3.

**[Table 2]**

| SEQ ID NO. | Sequence name | Sequence description |
|---|---|---|
| 1 | Antibody fragment 1 nucleotide | Nucleotide sequence encoding antibody fragment 1 |
| 2 | Antibody fragment 1 amino acid | Amino acid sequence corresponding to SEQ ID NO: 1 |
| 3 | Antibody fragment 2 nucleotide | Nucleotide sequence encoding antibody fragment 2 |
| 4 | Antibody fragment 2 amino acid | Amino acid sequence corresponding to SEQ ID NO: 3 |
| 5 | Antibody fragment 3 nucleotide | Nucleotide sequence encoding antibody fragment 3 |
| 6 | Antibody fragment 3 amino acid | Amino acid sequence corresponding to SEQ ID NO: 5 |
| 7 | CD8α LS nucleotide | Nucleotide sequence encoding CD8α-derived LS |
| 8 | CD8α LS amino acid | Amino acid sequence of CD8α-derived LS |
| 9 | CD8α hinge nucleotide | Nucleotide sequence encoding CD8α-derived hinge |
| 10 | CD8α hinge amino acid | Amino acid sequence of CD8α CD8α-derived hinge |
| 11 | CD8α TM nucleotide | Nucleotide sequence encoding CD8α-derived TM domain |
| 12 | CD8α TM amino acid | Amino acid sequence of CD8α-derived TM domain |
| 13 | 4-1BB SD nucleotide | Nucleotide sequence encoding 4-1BB-derived intracellular signaling domain (SD) |
| 14 | 4-1BB SD amino acid | Amino acid sequence of 4-1BB-derived intracellular SD |
| 15 | CD3ζ nucleotide | Nucleotide sequence encoding CD3ζ-derived intracellular SD |
| 16 | CD3ζ amino acid | Amino acid sequence of CD3ζ-derived intracellular SD |
| 17 | CAR 1 nucleotide | Nucleotide sequence encoding CAR 1 in Table 1 |
| 18 | CAR 1 amino acid | Amino acid sequence of CAR 1 in Table 1 |
| 19 | CAR 2 nucleotide | Nucleotide sequence encoding CAR 2 in Table 1 |
| 20 | CAR 2 amino acid | Amino acid sequence of CAR 2 in Table 1 |
| 21 | CAR 3 nucleotide | Nucleotide sequence encoding CAR 3 in Table 1 |
| 22 | CAR 3 amino acid | Amino acid sequence of CAR 3 in Table 1 |
| 23 | Linker nucleotide | Nucleotide sequence encoding a linker connecting VH and VL |
| 24 | Linker amino acid | Amino acid sequence of a linker connecting VH and VL |

**[Table 3]**

| SEQ ID NO. | Sequence name | Sequence |
|---|---|---|
| 1 | Antibody fragment 1 nucleotides | GAAGTACAACTGCTGGAAAGTGGCGGGGGTCTGGTTCAGCCGGGAGGCT CACTTAGGTTGTCATGTGCGGCATCCGGATTTACGTTCTCCGATTATGCGA TGTCCTGGGTTCGGCAAGCTCCTGGTAAAGGTTTGGAGTGGGTATCCGTA ATTTCTCATGATGGAGGCAGTATCTATTATGCCGATAGCGTCAAGGGACG CTTCACTATTTCCAGAGACAATTCCAAAAATACCCTGTACCTTCAAATGA ACTCACTCCGAGCCGAGGACACCGCAGTTTACTACTGCGCGAAGGGGCCT AGGAGAATCGCGAACGCTATTTTCGATTATTGGGGTCAGGGGACCCTGGT GACGGTTAGTTCTGGGGGTGGAGGCAGTGGAGGCGGGGGTAGTGGAGGA GGGGGTTCACAATTTGTACTGACCCAACCTCCGTCAGTCTCTGCCGCCCCC GGCCAAAAAGTAACGATAAGCTGTTCAGGGAGCAACTCTAATATAGGCA ACTCTTATGTCTCTTGGTACCAACAAGTACCCGGCGCAGCGCCTAGGCTTT TGATATATGGGTCAACTAACCGGCCGAGCGGCGTGCCAGACCGCTTTTCA GGTAGTAAGTCAGGGACGAGTGCCAGTCTGGCTATCACCGGTCTGCAAGC TGAGGACGAGGCTGATTATTACTGTCAAAGCTATGACTCCTCATTGTCCG GCTATGTTTTTGGAACGGGCACGAAGGTAACAGTTCTA |
| 2 | Antibody fragment 1 amino acids | EVQLLESGGGLVQPGGSLRLSCAASGFTFSDYAMSWVRQAPGKGLEWVSVI SHDGGSIYYADSVKGRFTISRDNSKNTLYLQMNSLRAEDTAVYYCAKGPRRI ANAIFDYWGQGTLVTVSSGGGGSGGGGSGGGGSQFVLTQPPSVSAAPGQKV TISCSGSNSNIGNSYVSWYQQVPGAAPRLLIYGSTNRPSGVPDRFSGSKSGTSA SLAITGLQAEDEADYYCQSYDSSLSGYVFGTGTKVTVL |
| 3 | Antibody fragment 2 nucleotides | GAGGTTCAGTTGCTTGAGTCAGGCGGAGGACTGGTTCAACCGGGCGGTAG TTTGAGGCTGAGCTGCGCCGCCAGTGGATTCACTTTCAGCGATTACGCGA TGTCCTGGGTCCGGCAGGCTCCGGGAAAGGGCCTGGAGTGGGTTTCTGTC ATAAGCCACGATGGCGACAGGGTATATTATGCTGACTCAGTGAAAGGTCG CTTCACAATTTCTCGGGACAACTCTAAAAATACCCTGTATTTGCAGATGA ATTCTCTCAGAGCGGAGGACACCGCTGTTTATTATTGTGCAAAAGGCCCC CGGAGGATCGCGAATGCCATATTCGATTACTGGGGCCAAGGCACGCTTGT AACGGTTTCATCAGGTGGAGGAGGGAGCGGTGGAGGCGGGTCCGGTGGC GGTGGATCTCAGCTCGTACTCACTCAGCCTCCGTCAGTATCTGGGGCCCC GGGTCAAAAGGTCACAATAAGTTGTTCCGGTTCTTCTAGTAATATCGGGC ATAGTTATGTCTCATGGTACCAGCAGTTGCCTGGAACTGCACCAAAGCTC CTGATATATGGGGATAACAATCGGCCTAGCGGTGTACCCGACCGCTTTAG CGGTTCTAAATCAGGCACATCTGCGTCCCTTGCTATTACCGGACTCCAGGC CGAAGACGAAGCGGACTATTACTGTCAGTCCTACGACAGTTCCTTGTCTG GGTATGTGTTCGGGACCGGAACCAAAGTCACTGTCCTA |
| 4 | Antibody fragment 2 amino acids | EVQLLESGGGLVQPGGSLRLSCAASGFTFSDYAMSWVRQAPGKGLEWVSVI SHDGDRVYYADSVKGRFTISRDNSKNTLYLQMNSLRAEDTAVYYCAKGPRR IANAIFDYWGQGTLVTVSSGGGGSGGGGSGGGGSQLVLTQPPSVSGAPGQK VTISCSGSSSNIGHSYVSWYQQLPGTAPKLLIYGDNNRPSGVPDRFSGSKSGTS ASLAITGLQAEDEADYYCQSYDSSLSGYVFGTGTKVTVL |
| 5 | Antibody fragment 3 nucleotides | GAAGTGCAGCTTTTGGAATCTGGCGGCGGACTCGTTCAGCCAGGAGGTTC CCTTAGACTGTCATGCGCGGCTAGCGGGTTTACGTTTAGCGACTATTCAAT GTCTTGGGTTCGACAGGCACCGGGCAAAGGTCTGGAGTGGGTGAGCGCA ATTAGCCCGGATCGGTCTCTGGAGTATTACGCCGACTCTGTGAAGGGGCG ATTTACCATCAGCCGGGACAACTCCAAAAATACCCTGTATCTGCAAATGA ACTCACTGCGAGCGGAAGACACCGCAGTCTACTATTGCGCAAAAGGGCC ACGCCATCTGACGAATAATATATTTGACTATTGGGGTCAAGGTACCCTCG TCACAGTTAGCTCAGGTGGCGGCGGTTCCGGCGGCGGTGGTAGCGGTGGG GGAGGCTCCCAATTCGTCTTGACCCAGCCACCAAGCGTGAGTGCTGCCCC CGGTCAGAAGGTCACCATATCTTGTAGTGGCTCAAGTTCAAACATAGGTC ACTCTTATGTTTCATGGTATCAACAACTCCCTGGAACGGCACCAAAACTTT TGATCTATGGTAACATTCACCGACCGTCCGGAGTACCTGATAGATTTAGC GGTTCTACGTCTGGGACTTCCGCTTCTCTTGCTATAACGGGCTTGCAGGCG GATGACGAGGCCGACTACTACTGTGGTGTGTGGGATTCTTCTCTGTCAGC GGTAGTGTTTGGAGGCGGGACAAAATTGACTGTACTA |
| 6 | Antibody fragment 3 amino acids | EVQLLESGGGLVQPGGSLRLSCAASGFTFSDYSMSWVRQAPGKGLEWVSAIS PDRSLEYYADSVKGRFTISRDNSKNTLYLQMNSLRAEDTAVYYCAKGPRHLT NNIFDYWGQGTLVTVSSGGGGSGGGGSGGGGSQFVLTQPPSVSAAPGQKVTI SCSGSSSNIGHSYVSWYQQLPGTAPKLLIYGNIHRPSGVPDRFSGSTSGTSASL AITGLQADDEADYYCGVWDSSLSAVVFGGGTKLTVL |
| 7 | CD8α LS nucleotides | ATGGCCTTACCAGTGACCGCCTTGCTCCTGCCGCTGGCCTTGCTGCTCCAC GCCGCCAGGCCG |
| 8 | CD8α LS amino acids | MALPVTALLLPLALLLHAARP |
| 9 | CD8α hinge nucleotides | ACCACGACGCCAGCGCCGCGACCACCAACACCGGCGCCCACCATCGCGTC GCAGCCCCTGTCCCTGCGCCCAGAGGCGTGCCGGCCAGCGGCGGGGGGC GCAGTGCACACGAGGGGGCTGGACTTCGCCTGTGAT |
| 10 | CD8α hinge amino acids | TTTPAPRPPTPAPTIASQPLSLRPEACRPAAGGAVHTRGLDFACD |
| 11 | CD8α TM nucleotides | ATCTACATCTGGGCGCCCTTGGCCGGGACTTGTGGGGTCCTTCTCCTGTCA CTGGTTATCACCCTTTACTGC |
| 12 | CD8α TM amino acids | IYIWAPLAGTCGVLLLSLVITLYC |
| 13 | 4-1BB SD nucleotides | AAACGGGGCAGAAAGAAACTCCTGTATATATTCAAACAACCATTTATGAG ACCAGTACAAACTACTCAAGAGGAAGATGGCTGTAGCTGCCGATTTCCAG AAGAAGAAGAAGGAGGATGTGAACTG |
| 14 | 4-1BB SD amino acids | KRGRKKLLYIFKQPFMRPVQTTQEEDGCSCRFPEEEEGGCEL |
| 15 | CD3ζ nucleotides | AGAGTGAAGTTCAGCAGGAGCGCAGACGCCCCCGCGTACAAGCAGGGCC AGAACCAGCTCTATAACGAGCTCAATCTAGGACGAAGAGAGGAGTACGA TGTTTTGGACAAGAGACGTGGCCGGGACCCTGAGATGGGGGGAAAGCCG AGAAGGAAGAACCCTCAGGAAGGCCTGTACAATGAACTGCAGAAAGATA AGATGGCGGAGGCCTACAGTGAGATTGGGATGAAAGGCGAGCGCCGGAG GGGCAAGGGGCACGATGGCCTTTACCAGGGTCTCAGTACAGCCACCAAG GACACCTACGACGCCCTTCACATGCAGGCCCTGCCCCCTCGC RVKFSRSADAPAYKQGQNQLYNELNLGRREEYDVLDKRRGRDPEMGGKPR RKNPQEGLYNELQKDKMAEAYSEIGMKGERRRGKGHDGLYQGLSTATKDT |
| 16 | CD3ζ amino acids | RVKFSRSADAPAYKQGQNQLYNELNLGRREEYDVLDKRRGRDPEMGGKPR RKNPQEGLYNELQKDKMAEAYSEIGMKGERRRGKGHDGLYQGLSTATKDT YDALHMQALPPR |
| 17 | CAR 1 nucleotides | ATGGCCTTACCAGTGACCGCCTTGCTCCTGCCGCTGGCCTTGCTGCTCCAC GCCGCCAGGCCGGAAGTACAACTGCTGGAAAGTGGCGGGGGTCTGGTTC AGCCGGGAGGCTCACTTAGGTTGTCATGTGCGGCATCCGGATTTACGTTC TCCGATTATGCGATGTCCTGGGTTCGGCAAGCTCCTGGTAAAGGTTTGGA GTGGGTATCCGTAATTTCTCATGATGGAGGCAGTATCTATTATGCCGATA GCGTCAAGGGACGCTTCACTATTTCCAGAGACAATTCCAAAAATACCCTG TACCTTCAAATGAACTCACTCCGAGCCGAGGACACCGCAGTTTACTACTG CGCGAAGGGGCCTAGGAGAATCGCGAACGCTATTTTCGATTATTGGGGTC AGGGGACCCTGGTGACGGTTAGTTCTGGGGGTGGAGGCAGTGGAGGCGG GGGTAGTGGAGGAGGGGGTTCACAATTTGTACTGACCCAACCTCCGTCAG TCTCTGCCGCCCCCGGCCAAAAAGTAACGATAAGCTGTTCAGGGAGCAAC TCTAATATAGGCAACTCTTATGTCTCTTGGTACCAACAAGTACCCGGCGC AGCGCCTAGGCTTTTGATATATGGGTCAACTAACCGGCCGAGCGGCGTGC CAGACCGCTTTTCAGGTAGTAAGTCAGGGACGAGTGCCAGTCTGGCTATC ACCGGTCTGCAAGCTGAGGACGAGGCTGATTATTACTGTCAAAGCTATGA CTCCTCATTGTCCGGCTATGTTTTTGGAACGGGCACGAAGGTAACAGTTCT AACCACGACGCCAGCGCCGCGACCACCAACACCGGCGCCCACCATCGCG TCGCAGCCCCTGTCCCTGCGCCCAGAGGCGTGCCGGCCAGCGGCGGGGGG CGCAGTGCACACGAGGGGGCTGGACTTCGCCTGTGATATCTACATCTGGG CGCCCTTGGCCGGGACTTGTGGGGTCCTTCTCCTGTCACTGGTTATCACCC TTTACTGCAAACGGGGCAGAAAGAAACTCCTGTATATATTCAAACAACCA TTTATGAGACCAGTACAAACTACTCAAGAGGAAGATGGCTGTAGCTGCCG ATTTCCAGAAGAAGAAGAAGGAGGATGTGAACTGAGAGTGAAGTTCAGC AGGAGCGCAGACGCCCCCGCGTACAAGCAGGGCCAGAACCAGCTCTATA ACGAGCTCAATCTAGGACGAAGAGAGGAGTACGATGTTTTGGACAAGAG ACGTGGCCGGGACCCTGAGATGGGGGGAAAGCCGAGAAGGAAGAACCCT CAGGAAGGCCTGTACAATGAACTGCAGAAAGATAAGATGGCGGAGGCCT ACAGTGAGATTGGGATGAAAGGCGAGCGCCGGAGGGGCAAGGGGCACG ATGGCCTTTACCAGGGTCTCAGTACAGCCACCAAGGACACCTACGACGCC CTTCACATGCAGGCCCTGCCCCCTCGCTAA |
| 18 | CAR 1 amino acids | MALPVTALLLPLALLLHAARPEVQLLESGGGLVQPGGSLRLSCAASGFTFSD YAMSWVRQAPGKGLEWVSVISHDGGSIYYADSVKGRFTISRDNSKNTLYLQ MNSLRAEDTAVYYCAKGPRRIANAIFDYWGQGTLVTVSSGGGGSGGGGSGG GGSQFVLTQPPSVSAAPGQKVTISCSGSNSNIGNSYVSWYQQVPGAAPRLLIY GSTNRPSGVPDRFSGSKSGTSASLAITGLQAEDEADYYCQSYDSSLSGYVFGT GTKVTVLTTTPAPRPPTPAPTIASQPLSLRPEACRPAAGGAVHTRGLDFACDIY IWAPLAGTCGVLLLSLVITLYCKRGRKKLLYIFKQPFMRPVQTTQEEDGCSCR FPEEEEGGCELRVKFSRSADAPAYKQGQNQLYNELNLGRREEYDVLDKRRG RDPEMGGKPRRKNPQEGLYNELQKDKMAEAYSEIGMKGERRRGKGHDGLY QGLSTATKDTYDALHMQALPPR |
| 19 | CAR 2 nucleotides | ATGGCCTTACCAGTGACCGCCTTGCTCCTGCCGCTGGCCTTGCTGCTCCAC GCCGCCAGGCCGGAGGTTCAGTTGCTTGAGTCAGGCGGAGGACTGGTTCA ACCGGGCGGTAGTTTGAGGCTGAGCTGCGCCGCCAGTGGATTCACTTTCA GCGATTACGCGATGTCCTGGGTCCGGCAGGCTCCGGGAAAGGGCCTGGA GTGGGTTTCTGTCATAAGCCACGATGGCGACAGGGTATATTATGCTGACT CAGTGAAAGGTCGCTTCACAATTTCTCGGGACAACTCTAAAAATACCCTG TATTTGCAGATGAATTCTCTCAGAGCGGAGGACACCGCTGTTTATTATTGT GCAAAAGGCCCCCGGAGGATCGCGAATGCCATATTCGATTACTGGGGCCA AGGCACGCTTGTAACGGTTTCATCAGGTGGAGGAGGGAGCGGTGGAGGC GGGTCCGGTGGCGGTGGATCTCAGCTCGTACTCACTCAGCCTCCGTCAGT ATCTGGGGCCCCGGGTCAAAAGGTCACAATAAGTTGTTCCGGTTCTTCTA GTAATATCGGGCATAGTTATGTCTCATGGTACCAGCAGTTGCCTGGAACT GCACCAAAGCTCCTGATATATGGGGATAACAATCGGCCTAGCGGTGTACC CGACCGCTTTAGCGGTTCTAAATCAGGCACATCTGCGTCCCTTGCTATTAC CGGACTCCAGGCCGAAGACGAAGCGGACTATTACTGTCAGTCCTACGACA GTTCCTTGTCTGGGTATGTGTTCGGGACCGGAACCAAAGTCACTGTCCTA ACCACGACGCCAGCGCCGCGACCACCAACACCGGCGCCCACCATCGCGTC GCAGCCCCTGTCCCTGCGCCCAGAGGCGTGCCGGCCAGCGGCGGGGGGC GCAGTGCACACGAGGGGGCTGGACTTCGCCTGTGATATCTACATCTGGGC GCCCTTGGCCGGGACTTGTGGGGTCCTTCTCCTGTCACTGGTTATCACCCT TTACTGCAAACGGGGCAGAAAGAAACTCCTGTATATATTCAAACAACCAT TTATGAGACCAGTACAAACTACTCAAGAGGAAGATGGCTGTAGCTGCCGA TTTCCAGAAGAAGAAGAAGGAGGATGTGAACTGAGAGTGAAGTTCAGCA GGAGCGCAGACGCCCCCGCGTACAAGCAGGGCCAGAACCAGCTCTATAA CGAGCTCAATCTAGGACGAAGAGAGGAGTACGATGTTTTGGACAAGAGA CGTGGCCGGGACCCTGAGATGGGGGGAAAGCCGAGAAGGAAGAACCCTC AGGAAGGCCTGTACAATGAACTGCAGAAAGATAAGATGGCGGAGGCCTA CAGTGAGATTGGGATGAAAGGCGAGCGCCGGAGGGGCAAGGGGCACGAT GGCCTTTACCAGGGTCTCAGTACAGCCACCAAGGACACCTACGACGCCCT TCACATGCAGGCCCTGCCCCCTCGCTAA |
| 20 | CAR 2 amino acids | MALPVTALLLPLALLLHAARPEVQLLESGGGLVQPGGSLRLSCAASGFTFSD YAMSWVRQAPGKGLEWVSVISHDGDRVYYADSVKGRFTISRDNSKNTLYLQ MNSLRAEDTAVYYCAKGPRRIANAIFDYWGQGTLVTVSSGGGGSGGGGSGG GGSQLVLTQPPSVSGAPGQKVTISCSGSSSNIGHSYVSWYQQLPGTAPKLLIY GDNNRPSGVPDRFSGSKSGTSASLAITGLQAEDEADYYCQSYDSSLSGYVFG TGTKVTVLTTTPAPRPPTPAPTIASQPLSLRPEACRPAAGGAVHTRGLDFACDI YIWAPLAGTCGVLLLSLVITLYCKRGRKKLLYIFKQPFMRPVQTTQEEDGCSC RFPEEEEGGCELRVKFSRSADAPAYKQGQNQLYNELNLGRREEYDVLDKRR GRDPEMGGKPRRKNPQEGLYNELQKDKMAEAYSEIGMKGERRRGKGHDGL YQGLSTATKDTYDALHMQALPPR |
| 21 | CAR 3 nucleotides | ATGGCCTTACCAGTGACCGCCTTGCTCCTGCCGCTGGCCTTGCTGCTCCAC GCCGCCAGGCCGGAAGTGCAGCTTTTGGAATCTGGCGGCGGACTCGTTCA GCCAGGAGGTTCCCTTAGACTGTCATGCGCGGCTAGCGGGTTTACGTTTA GCGACTATTCAATGTCTTGGGTTCGACAGGCACCGGGCAAAGGTCTGGAG TGGGTGAGCGCAATTAGCCCGGATCGGTCTCTGGAGTATTACGCCGACTC TGTGAAGGGGCGATTTACCATCAGCCGGGACAACTCCAAAAATACCCTGT ATCTGCAAATGAACTCACTGCGAGCGGAAGACACCGCAGTCTACTATTGC GCAAAAGGGCCACGCCATCTGACGAATAATATATTTGACTATTGGGGTCA AGGTACCCTCGTCACAGTTAGCTCAGGTGGCGGCGGTTCCGGCGGCGGTG GTAGCGGTGGGGGAGGCTCCCAATTCGTCTTGACCCAGCCACCAAGCGTG AGTGCTGCCCCCGGTCAGAAGGTCACCATATCTTGTAGTGGCTCAAGTTC AAACATAGGTCACTCTTATGTTTCATGGTATCAACAACTCCCTGGAACGG CACCAAAACTTTTGATCTATGGTAACATTCACCGACCGTCCGGAGTACCT GATAGATTTAGCGGTTCTACGTCTGGGACTTCCGCTTCTCTTGCTATAACG GGCTTGCAGGCGGATGACGAGGCCGACTACTACTGTGGTGTGTGGGATTC TTCTCTGTCAGCGGTAGTGTTTGGAGGCGGGACAAAATTGACTGTACTAA CCACGACGCCAGCGCCGCGACCACCAACACCGGCGCCCACCATCGCGTCG CAGCCCCTGTCCCTGCGCCCAGAGGCGTGCCGGCCAGCGGCGGGGGGCG CAGTGCACACGAGGGGGCTGGACTTCGCCTGTGATATCTACATCTGGGCG CCCTTGGCCGGGACTTGTGGGGTCCTTCTCCTGTCACTGGTTATCACCCTT TACTGCAAACGGGGCAGAAAGAAACTCCTGTATATATTCAAACAACCATT TATGAGACCAGTACAAACTACTCAAGAGGAAGATGGCTGTAGCTGCCGAT TTCCAGAAGAAGAAGAAGGAGGATGTGAACTGAGAGTGAAGTTCAGCAG GAGCGCAGACGCCCCCGCGTACAAGCAGGGCCAGAACCAGCTCTATAAC GAGCTCAATCTAGGACGAAGAGAGGAGTACGATGTTTTGGACAAGAGAC GTGGCCGGGACCCTGAGATGGGGGGAAAGCCGAGAAGGAAGAACCCTCA GGAAGGCCTGTACAATGAACTGCAGAAAGATAAGATGGCGGAGGCCTAC AGTGAGATTGGGATGAAAGGCGAGCGCCGGAGGGGCAAGGGGCACGATG GCCTTTACCAGGGTCTCAGTACAGCCACCAAGGACACCTACGACGCCCTT CACATGCAGGCCCTGCCCCCTCGCTAA |
| 22 | CAR 3 amino acids | MALPVTALLLPLALLLHAARPEVQLLESGGGLVQPGGSLRLSCAASGFTFSD YSMSWVRQAPGKGLEWVSAISPDRSLEYYADSVKGRFTISRDNSKNTLYLQ MNSLRAEDTAVYYCAKGPRHLTNNIFDYWGQGTLVTVSSGGGGSGGGGSG GGGSQFVLTQPPSVSAAPGQKVTISCSGSSSNIGHSYVSWYQQLPGTAPKLLI YGNIHRPSGVPDRFSGSTSGTSASLAITGLQADDEADYYCGVWDSSLSAVVF GGGTKLTVLTTTPAPRPPTPAPTIASQPLSLRPEACRPAAGGAVHTRGLDFAC DIYIWAPLAGTCGVLLLSLVITLYCKRGRKKLLYIFKQPFMRPVQTTQEEDGC SCRFPEEEEGGCELRVKFSRSADAPAYKQGQNQLYNELNLGRREEYDVLDK RRGRDPEMGGKPRRKNPQEGLYNELQKDKMAEAYSEIGMKGERRRGKGHD GLYQGLSTATKDTYDALHMQALPPR |
| 23 | Linker nucleotides | GGGGGTGGAGGCAGTGGAGGCGGGGGTAGTGGAGGAGGGGGTTCA |
| 24 | Linker amino acids | GGGGSGGGGSGGGGS |

The sequence lists of the heavy chain, light chain, and CDRs of the antibody fragment variable regions used in the production of the CARs according to an example of the present invention are summarized in Tables 4 and 5.

**[Table 4]**

| Clone | SEQ ID NO. | Variable region | Amino acid sequence |
|---|---|---|---|
| Antibody fragment 1 | 25 | Heavy chain | EVQLLESGGGLVQPGGSLRLSCAASGFTFSDYAMSWVRQAPGKGLE WVSVISHDGGSIYYADSVKGRFTISRDNSKNTLYLQMNSLRAEDTA VYYCAKGPRRIANAIFDYWGQGTLVTVSS |
| | 26 | Light chain | QFVLTQPPSVSAAPGQKVTISCSGSNSNIGNSYVSWYQQVPGAAPRL LIYGSTNRPSGVPDRFSGSKSGTSASLAITGLQAEDEADYYCQSYDSS LSGYVFGTGTKVTVL |
| Antibody fragment 2 | 27 | Heavy chain | EVQLLESGGGLVQPGGSLRLSCAASGFTFSDYAMSWVRQAPGKGLE WVSVISHDGDRVYYADSVKGRFTISRDNSKNTLYLQMNSLRAEDTA VYYCAKGPRRIANAIFDYWGQGTLVTVSS |
| | 28 | Light chain | QLVLTQPPSVSGAPGQKVTISCSGSSSNIGHSYVSWYQQLPGTAPKL LIYGDNNRPSGVPDRFSGSKSGTSASLAITGLQAEDEADYYCQSYDS SLSGYVFGTGTKVTVL |
| Antibody fragment 3 | 29 | Heavy chain | EVQLLESGGGLVQPGGSLRLSCAASGFTFSDYSMSWVRQAPGKGLE WVSAISPDRSLEYYADSVKGRFTISRDNSKNTLYLQMNSLRAEDTA VYYCAKGPRHLTNNIFDYWGQGTLVTVSS |
| | 30 | Light chain | QFVLTQPPSVSAAPGQKVTISCSGSSSNIGHSYVSWYQQLPGTAPKLL IYGNIHRPSGVPDRFSGSTSGTSASLAITGLQADDEADYYCGVWDSS LSAVVFGGGTKLTVL |

**[Table 5]**

| Clone | Variable region | SEQ ID NO. | CDR1 | SEQ ID NO. | CDR2 | SEQ ID NO. | CDR3 |
|---|---|---|---|---|---|---|---|
| Antibody | Heavy | 31 | DYAMS | 32 | VISHDGGSIY | 33 | GPRRIANAIFDY |
| fragment 1 | chain | | | | YADSVKG | | |
| | Light chain | 34 | SGSNSNIGN SYVS | 35 | GSTNRPS | 36 | QSYDSSLSGYV |
| Antibody fragment 2 | Heavy chain | 37 | DYAMS | 38 | VISHDGDRVY YADSVKG | 39 | GPRRIANAIFDY |
| | Light chain | 40 | SGSSSNIGHS YVS | 41 | GDNNRPS | 42 | QSYDSSLSGYV |
| Antibody fragment 3 | Heavy chain | 43 | DYSMS | 44 | AISPDRSLEYY ADSVKG | 45 | GPRHL TNNIFDY |
| | Light chain | 46 | SGSSSNIGHS YVS | 47 | GNIHRPS | 48 | GVWDSSLSAVV |

### Example 4: Production of anti-HLA-G CAR-T cells

### 4-1. Production of anti-HLA-G CAR gene-expressing lentivirus

Lentiviruses for delivery of the anti-HLA-G CAR gene were produced by plasmid DNA transfection. The TransIT-293 transfection reagent (Mirus Bio LLC, USA) was used, and transfection was performed according to the manufacturer's protocol. 5×10⁶ cells of the Lenti-X^{™} 293T cell line (Clontech, USA) were seeded in a 100 mm dish the day before the experiment, and a pLV-EF1A-HLA-G CAR lentiviral vector, a gag-pol expression vector, and a VSV-G envelope expression vector were transfected the next day. HLA-G CAR lentiviral vectors are as follows; antibody fragment 1, antibody fragment 2, or antibody fragment 3. After the transfection, cells were cultured for approximately 72 hours, and all cell supernatants were harvested after the culture was completed. The supernatant was filtered through a 0.45 µm filter (Millipore, USA) to remove cell debris. Lentiviruses were concentrated approximately 100-fold using the Lenti-X^{™} concentrator (Clontech, USA) according to the manufacturer's protocol and stored frozen at -80 °C until use.

### 4-2. Production and culture of anti-HLA-G CAR-expressing T cells

Donors were recruited, and leukocytes were obtained through leukapheresis. Peripheral blood mononuclear cells (PBMCs) were obtained from the leukocytes using SepMate^{™}-50 (STEMCELL Technology, Canada) and Ficoll-Paque PLUS (GE Healthcare, Sweden). Thereafter, human T cells were separated by positive selection using QuadroMACS^{™} Separator (Miltenyi Biotec, Germany), a large-scale (LS) column (Miltenyi Biotec, Germany), human CD4 MicroBeads (Miltenyi Biotec, Germany), and human CD8 MicroBeads (Miltenyi Biotec, Germany). The human T cells were cultured in the TexMACS^{™} medium (Miltenyi Biotec, Germany) and activated by adding T Cell TransAct^{™}, human (Miltenyi Biotec, Germany). For the growth of T cells, human IL-2 (R&D Systems, USA) was added to the culture medium at 100 U/mL and cultured. After culturing for 48 hours, the activated T cells were harvested and used for lentivirus transduction.

Activated human T cells together with a lentivirus having a multiplicity of infection (MOI) of approximately 3 were placed into a 12-well plate at a density of 1×10⁶ per well, and the culture medium was prepared to be a volume of 2.5 mL. The lentivirus was transduced into the T cells by centrifugation at 1,000 xg for 15 minutes. After culturing the T cells by stationary culture for 48 hours, the culture medium was replaced with fresh medium. The transduced human T cells were subcultured at 5×10⁵ per mL at intervals of two to three days and maintained so that the cell number did not exceed 2×10⁶ per mL. Unless otherwise mentioned, the human T cells were cultured by adding 100 U/mL of IL-2 (R&D Systems, USA) to the culture medium.

### 4-3. Confirmation of CAR expression in anti-HLA-G CAR-expressing T cells

It was confirmed whether the anti-HLA-G CAR is expressed on the cell surface of anti-HLA-G CAR-expressing T cells generated from leukocytes obtained from three donors.

After harvesting 1×10⁵ cells, the cells were suspended in 100 µL of D-PBS to prepare a cell suspension. Phycoerythrin (PE) fluorescence was conjugated with the B2M-HLA-G antigen used for producing the anti-HLA-G antibodies, using a PE conjugation kit (Abcam, USA). The B2M-HLA-G antigen-PE conjugate was added to the cell suspension and allowed to react at 4 °C for 30 minutes. After the reaction was completed, the cells were washed twice with D-PBS, and the expression level of the anti-HLA-G CAR was confirmed by flow cytometry.

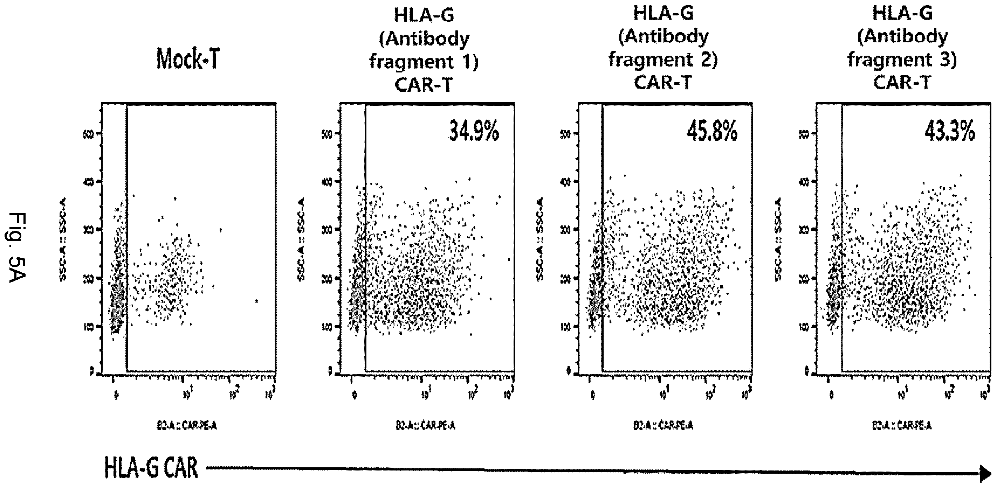

As a result, in Donor 1, it was confirmed that approximately 34.9% of the anti-HLA-G CAR was expressed on the surface of the T cells into which the anti-HLA-G CAR (antibody fragment 1 CAR) gene was transduced, approximately 45.8% of the anti-HLA-G CAR was expressed on the surface of the T cells into which the anti-HLA-G CAR (antibody fragment 2 CAR) gene was transduced, and approximately 43.3% of the anti-HLA-G CAR was expressed on the surface of the T cells into which the anti-HLA-G CAR (antibody fragment 3 CAR) gene was transduced (FIG. 5A).

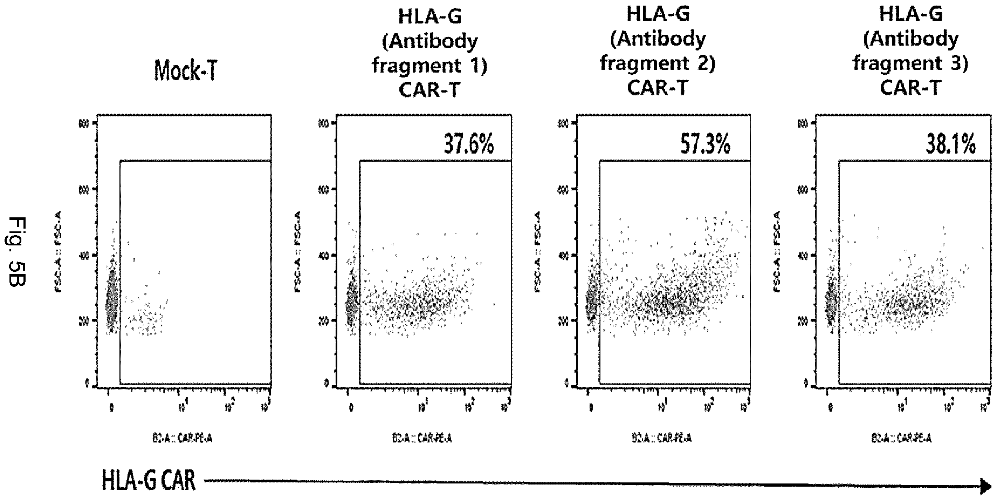

As a result, in Donor 2, it was confirmed that approximately 37.6% of the anti-HLA-G CAR was expressed on the surface of the T cells into which the anti-HLA-G CAR (antibody fragment 1 CAR) gene was transduced, approximately 57.3% of the anti-HLA-G CAR was expressed on the surface of the T cells into which the anti-HLA-G CAR (antibody fragment 2 CAR) gene was transduced, and approximately 38.1% of the anti-HLA-G CAR was expressed on the surface of the T cells into which the anti-HLA-G CAR (antibody fragment 3 CAR) gene was transduced (FIG. 5B).

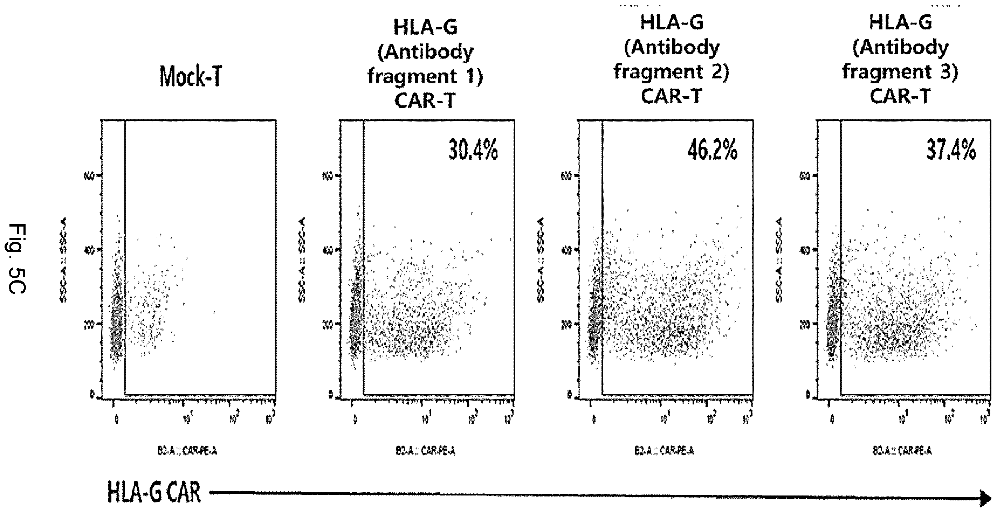

As a result, in Donor 5, it was confirmed that approximately 30.4% of the anti-HLA-G CAR was expressed on the surface of the T cells into which the anti-HLA-G CAR (antibody fragment 1 CAR) gene was transduced, approximately 46.2% of the anti-HLA-G CAR was expressed on the surface of the T cells into which the anti-HLA-G CAR (antibody fragment 2 CAR) gene was transduced, and approximately 37.4% of the anti-HLA-G CAR was expressed on the surface of the T cells into which the anti-HLA-G CAR (antibody fragment 3 CAR) gene was transduced (FIG. 5C).

### Example 5: Confirmation of in vitro anticancer efficacy of anti-HLA-G CAR-expressing T cells

The anticancer efficacy of the anti-HLA-G CAR-expressing T cells (effector cells, E) against target cells (T) was confirmed by the method described below.
(i) All target cells were produced to overexpress EGFP.
(ii) Target cells were added to a 48-well plate at 1×10⁵ cells/150 µL per well.
(iii) Anti-HLA-G CAR-expressing T cells and control T cells were added at 1×10⁶ cells/150 µL, 5×10⁵ cells/150 µL, and 1×10⁵ cells/150 µL per well (E:T ratio = 10, 5, 1) and allowed to react with the target cells for 24 hours.
(iv) Since the EGFP fluorescence signal disappears when target cells die, the number of cells expressing EGFP was counted using flow cytometry after 24 hours. Cytotoxicity (%)=100-{(Number of EGFP+ cells after co-culture of target cells and effector cells/Number of EGFP+ cells after culture of target cells alone) × 100}

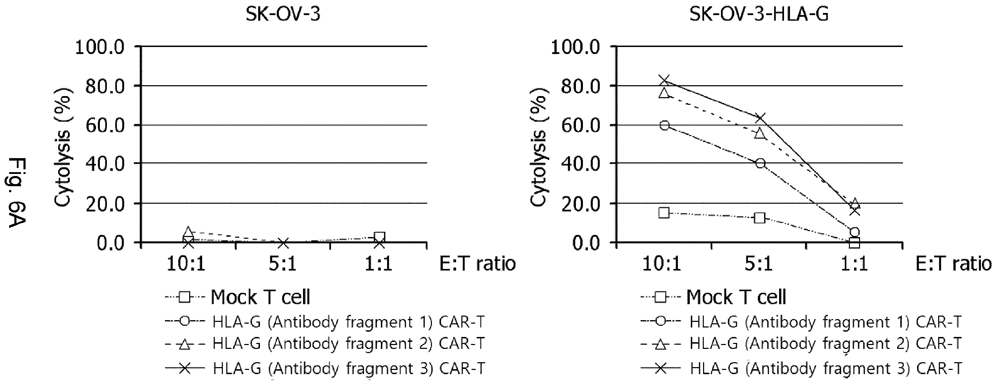

As a result of the experiment, the anticancer efficacy of the three types of anti-HLA-G CAR (antibody fragment 1 CAR), anti-HLA-G CAR (antibody fragment 2 CAR), and anti-HLA-G CAR (antibody fragment 3 CAR)-expressing T cells produced from the leukocytes of Donor 1 against the HLA-G-overexpressing SK-OV-3-HLA-G cell line was significantly higher than that of the control T cells that did not express anti-HLA-G CAR. However, there was no difference in killing ability between the three types of anti-HLA-G CAR-expressing T cells and the control T cells against the SK-OV-3 cell that did not express anti-HLA-G (FIG. 6A).

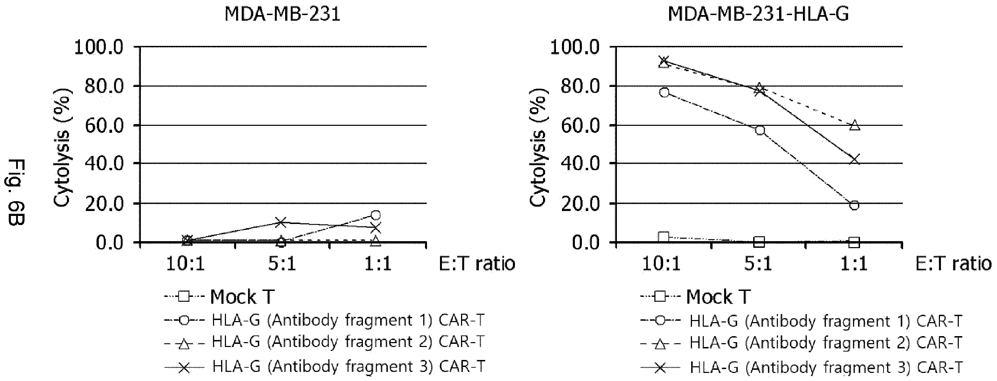

The anticancer efficacy of the three types of anti-HLA-G CAR (antibody fragment 1 CAR), anti-HLA-G CAR (antibody fragment 2 CAR), and anti-HLA-G CAR (antibody fragment 3 CAR)-expressing T cells produced from the leukocytes of Donor 2 against the HLA-G-overexpressing MDA-MB-231-HLA-G cell line was significantly higher than that of the control T cells that did not express anti-HLA-G CAR. On the other hand, there was no difference in killing ability between the three types of anti-HLA-G CAR-expressing T cells and the control T cells against the MDA-MB-231cell line that did not express anti-HLA-G (FIG. 6B).

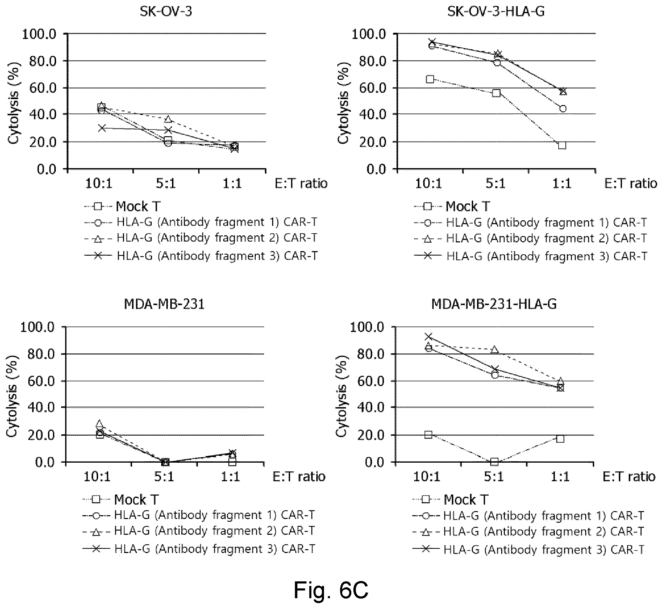

The anticancer efficacy of the three types of anti-HLA-G CAR (antibody fragment 1 CAR), anti-HLA-G CAR (antibody fragment 2 CAR), and anti-HLA-G CAR (antibody fragment 3 CAR)-expressing T cells produced from the leukocytes of Donor 5 against the HLA-G-overexpressing SK-OV-3-HLA-G and MDA-MB-231-HLA-G cell lines was also significantly higher than that of the control T cells that did not express anti-HLA-G CAR. However, there was no difference in killing ability between the three types of anti-HLA-G CAR-expressing T cells and the control T cells against the SK-OV-3 and MDA-MB-231 cell lines that did not express anti-HLA-G (FIG. 6C).

Through the above-described results, it was confirmed that the anti-HLA-G CAR-expressing T cells have high killing ability specifically only against cells that highly express HLA-G.

Cell supernatants were harvested from the experimental group in which the anti-HLA-G CAR-expressing T cells and control T cells produced from Donor 5 were treated with the target cells at an E:T ratio of 1:1. The concentration of interferon gamma in the harvested supernatants was measured using Human IFN-gamma Quantikine enzyme-linked immunosorbent assay (ELISA) Kit (R&D Systems, USA).

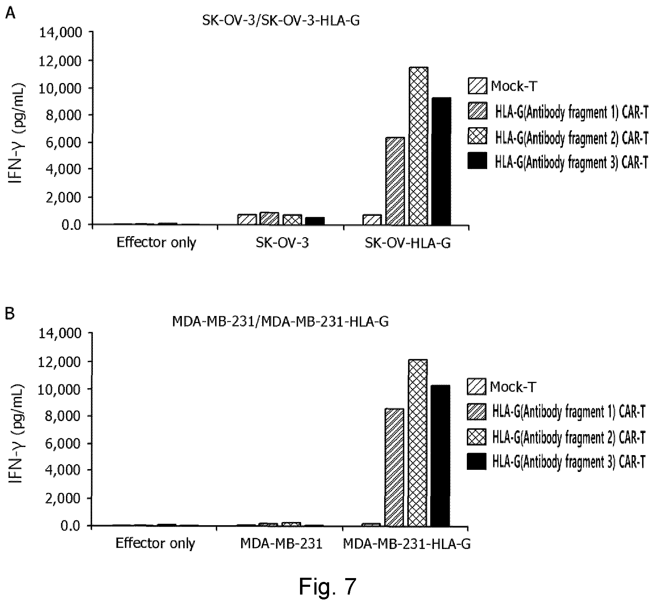

As a result, it was confirmed that the secretion of interferon gamma significantly increased only when the T cells expressing three types of anti-HLA-G CAR (antibody fragment 1 CAR), anti-HLA-G CAR (antibody fragment 2 CAR), and anti-HLA-G CAR (antibody fragment 3 CAR) were co-cultured with the HLA-G-overexpressing SK-OV-3-HLA-G or MDA-MB-231-HLA-G target cell line (FIG. 7).

### Example 6: Comparison of in vitro anticancer efficacy of anti-HLA-G CAR-expressing T cells according to T cell culture conditions

Anti-HLA-G CAR (antibody fragment 2 CAR)-expressing T cells were produced in the manner mentioned in Example 4-2. At this time, for the growth of T cells, human IL-2 (R&D Systems, USA) was added to the culture medium at 100 U/mL, or IL-7 (Miltenyi Biotec, Germany) and IL-15 (Miltenyi Biotec, Germany) were each added at 12.5 ng/mL, and then the cells were cultured.

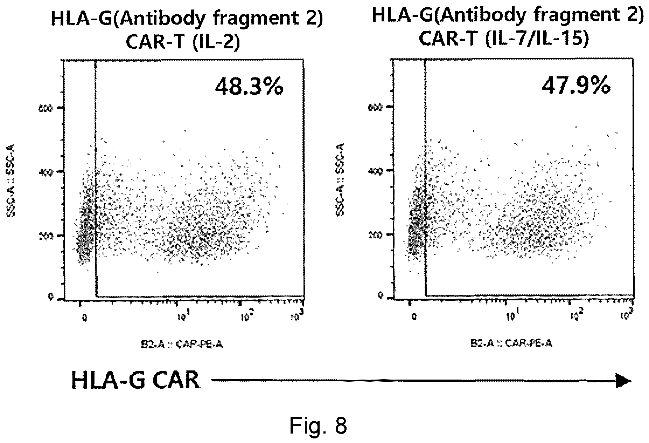

As a result of confirming whether the anti-HLA-G CAR is expressed on the cell surface of the anti-HLA-G CAR-expressing T cells, it was confirmed that approximately 48.3% of the anti-HLA-G CAR was expressed on the surface of the anti-HLA-G CAR-expressing T cells cultured by adding IL-2 to the culture medium, and approximately 47.9% of the anti-HLA-G CAR was expressed on the surface of the anti-HLA-G CAR-expressing T cells cultured by adding IL-7 and IL-15 to the culture medium (FIG. 8).

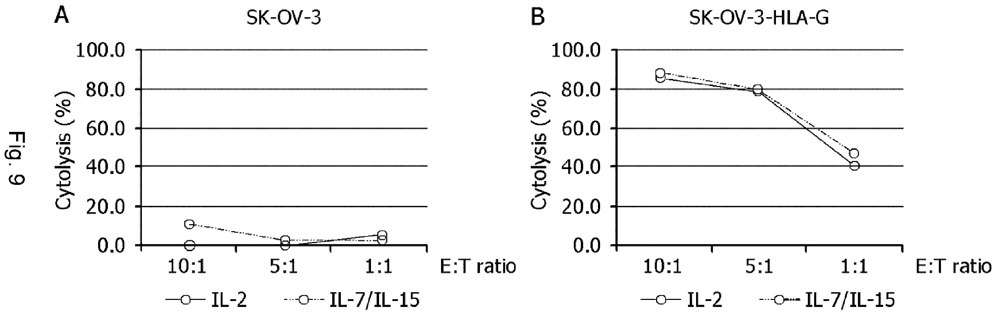

As a result of confirming the anticancer efficacy by co-culturing the anti-HLA-G CAR-expressing T cells with target cells, the anticancer efficacy of the anti-HLA-G CAR-expressing T cells (IL-2) and the anti-HLA-G CAR-expressing T cells (IL-7/IL-15) against the HLA-G-overexpressing SK-OV-3-HLA-G cell line was similarly excellent. On the other hand, the killing ability was significantly low against the SK-OV-3 cell line that did not express HLA-G, and thus there was no difference in killing ability between the two types of anti-HLA-G CAR-expressing T cells (FIG. 9).

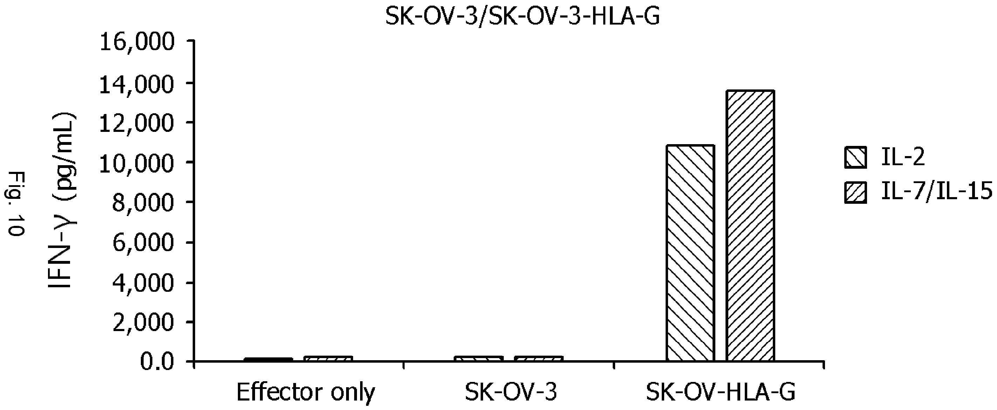

Cell supernatants were harvested from the experimental group in which the anti-HLA-G CAR-expressing T cells were treated with the target cells at an E:T ratio of 1:1, and the concentration of interferon gamma was measured using Human IFN-gamma Quantikine ELISA Kit (R&D Systems, USA). As a result, it was confirmed that the secretion of interferon gamma significantly increased only when the anti-HLA-G CAR-expressing T cells (IL-2) or the anti-HLA-G CAR-expressing T cells (IL-7/IL-15) were co-cultured with the HLA-G-overexpressing SK-OV-3-HLA-G target cell line, and there was almost no difference between the two types of anti-HLA-G CAR-expressing T cells (FIG. 10).

Through the above-described results, it was confirmed that the anti-HLA-G CAR-expressing T cells exhibited similar levels of CAR expression regardless of the type of cytokine (IL-2 or IL-7/IL-15) added to the culture medium for T cell growth, and both types of anti-HLA-G CAR-expressing T cells exhibited good anticancer efficacy *in vitro.*

### Example 7: Production of anti-HLA-G CAR-NK cells and confirmation of anticancer efficacy

### 7-1. Production and culture of anti-HLA-G CAR-expressing NK cells

Donors were recruited, and leukocytes were obtained through leukapheresis. PBMCs were obtained from the leukocytes using SepMate^{™}-50 (STEMCELL Technology, Canada) and Ficoll-Paque PLUS (GE Healthcare, Sweden). Thereafter, human NK cells were separated by negative selection using an NK cell isolation kit (Miltenyi Biotec, Germany). The human NK cells were cultured using the NK MACS^{®} medium (Miltenyi Biotec, Germany) as a culture solution. For efficient growth of the NK cells, the cells were cultured by adding 5% GemCell^{™} human serum AB (GeminiBio, USA) and human IL-2 (R&D Systems, USA) and IL-15 (Miltenyi Biotec, Germany) at concentrations of 500 U/mL and 140 U/mL to the culture solution, respectively.

RetroNectin^{®} (Takara, Japan) and BX-795 (Sigma-Aldrich, USA) were used to efficiently deliver lentivirus into NK cells. A lentivirus having an MOI of approximately 10 was placed into a RetroNectin-coated 24-well plate, and the lentivirus was adsorbed according to the manufacturer's protocol. 5×10⁵ NK cells on day 10 of the culture were treated with 2.5 µM BX-795 for 30 minutes. Thereafter, the NK cells treated with BX-795 were placed into the lentivirus-adsorbed plate, and centrifuged at 1,000 xg for 15 minutes to deliver the lentivirus into NK cells. Thereafter, the NK cells were cultured by stationary culture for 24 hours, and then the culture medium was replaced with fresh medium. The transduced human NK cells were appropriately subcultured at intervals of three to four days.

### 7-2. Confirmation of CAR expression in anti-HLA-G CAR-expressing NK cells

First, it was confirmed whether the anti-HLA-G CAR-expressing NK cells were properly cultured. 1×10⁵ cells were harvested and suspended in 100 µL of D-PBS to prepare a cell suspension. A VioBlue-conjugated CD3 antibody (Miltenyi Biotec, Germany) and FITC-conjugated CD56 antibody (Miltenyi Biotec, Germany) were added to the cell suspension and allowed to react at 4 °C for 30 minutes. After the reaction, the cells were washed twice with D-PBS, and the expression levels of CD3 and CD56 were confirmed by flow cytometry.

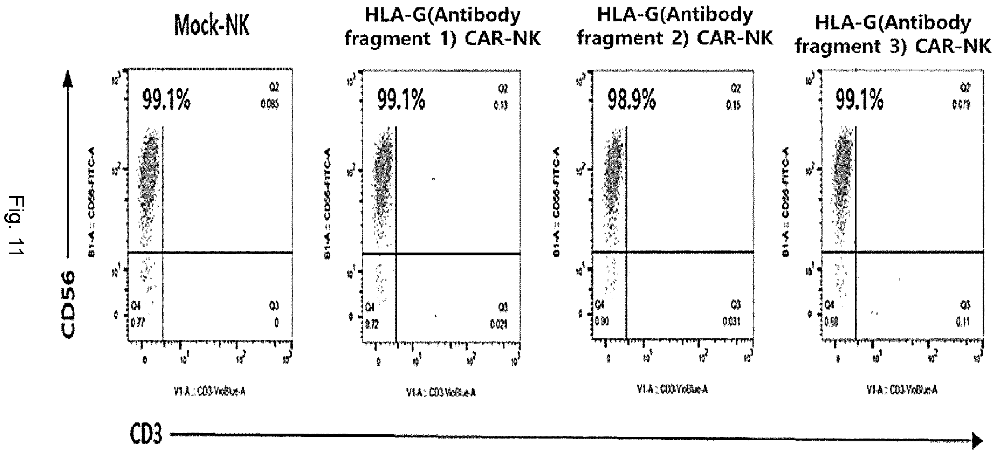

As a result, it was found that the proportion of the CD3⁻CD56⁺ NK cells was 99.1% in the control NK cells, 99.1% in the NK cells into which the anti-HLA-G CAR (antibody fragment 1 CAR) gene was transduced, 98.9% in the NK cells into which the anti-HLA-G CAR (antibody fragment 2 CAR) gene was transduced, and 99.1% in the NK cells into which the anti-HLA-G CAR (antibody fragment 3 CAR) gene was transduced, confirming that almost all cells were CD3⁻CD56⁺ NK cells (FIG. 11).

Next, it was confirmed whether the anti-HLA-G CAR was expressed on the surface of each cell. 1×10⁵ cells were harvested and suspended in 100 µL of D-PBS to prepare a cell suspension. PE fluorescence was conjugated with the B2M-HLA-G antigen used for producing anti-HLA-G antibodies, using a PE conjugation kit (Abcam, USA). The B2M-HLA-G antigen-PE conjugate was added to the cell suspension and allowed to react at 4 °C for 30 minutes. After the reaction was completed, the cells were washed twice with D-PBS, and the expression of the anti-HLA-G CAR was confirmed by flow cytometry.

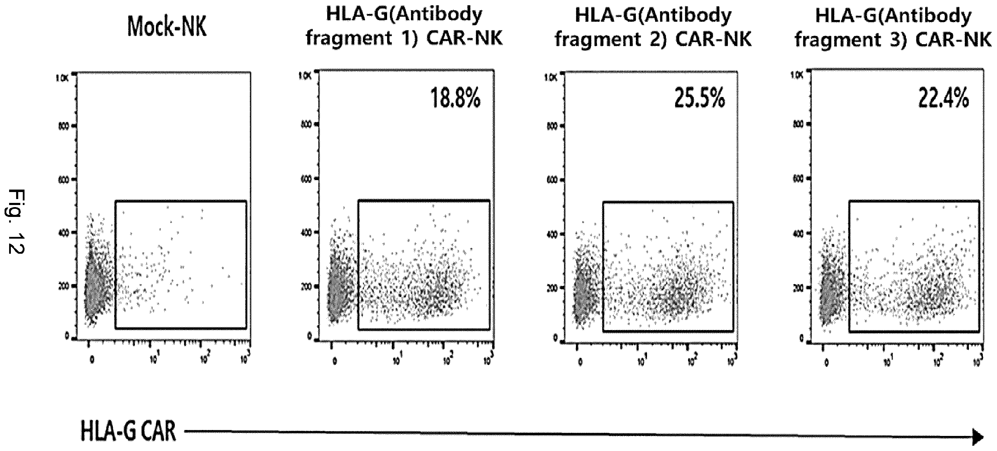

As a result, it was confirmed that approximately 18.8% of the anti-HLA-G CAR was expressed on the surface of the NK cells into which the anti-HLA-G CAR (antibody fragment 1 CAR) gene was transduced, approximately 25.5% of the anti-HLA-G CAR was expressed on the surface of the NK cells into which the anti-HLA-G CAR (antibody fragment 2 CAR) gene was transduced, and approximately 22.4% of the anti-HLA-G CAR was expressed on the surface of the NK cells into which the anti-HLA-G CAR (antibody fragment 3 CAR) gene was transduced (FIG. 12).

### 7-3. Confirmation of in vitro anticancer efficacy of anti-HLA-G CAR-expressing NK cells

The anticancer efficacy of the anti-HLA-G CAR expressing NK cells was confirmed in the same manner as in Example 5.

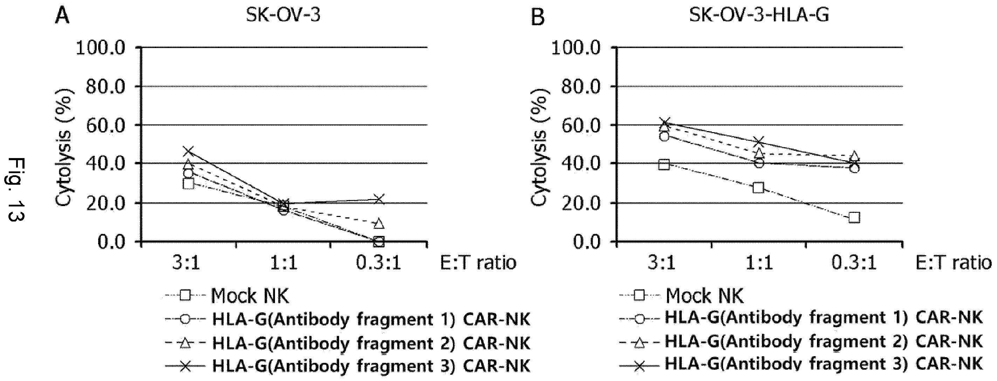

As a result, the anticancer efficacy of three types of anti-HLA-G CAR (antibody fragment 1 CAR), anti-HLA-G CAR (antibody fragment 2 CAR), and anti-HLA-G CAR (antibody fragment 3 CAR)-expressing NK cells against the HLA-G-overexpressing SK-OV-3-HLA-G cell line was significantly higher than that of the control NK cells that did not express the anti-HLA-G CAR. However, in the SK-OV-3 cell line, there was no difference in killing ability between the anti-HLA-G CAR-expressing NK cells and the control NK cells (FIG. 13).

## Claims

1. An anti-human leukocyte antigen-G (anti-HLA-G) chimeric antigen receptor (CAR) comprising:
an antigen-binding domain specifically binding to HLA-G;
a transmembrane domain; and
an intracellular signaling domain,
wherein the antigen-binding domain specifically binding to HLA-G includes a sequence selected from the following:
(a) a heavy chain variable region including a heavy chain complementarity determining region 1 (CDR1) having an amino acid sequence of SEQ ID NO: 31, a heavy chain CDR2 having an amino acid sequence of SEQ ID NO: 32, and a heavy chain CDR3 having an amino acid sequence of SEQ ID NO: 33; and
a light chain variable region including a light chain CDR1 having an amino acid sequence of SEQ ID NO: 34, a light chain CDR2 having an amino acid sequence of SEQ ID NO: 35, and a light chain CDR3 having an amino acid sequence of SEQ ID NO: 36;
(b) a heavy chain variable region including a heavy chain CDR1 having an amino acid sequence of SEQ ID NO: 37, a heavy chain CDR2 having an amino acid sequence of SEQ ID NO: 38, and a heavy chain CDR3 having an amino acid sequence of SEQ ID NO: 39; and
a light chain variable region including a light chain CDR1 having an amino acid sequence of SEQ ID NO: 40, a light chain CDR2 having an amino acid sequence of SEQ ID NO: 41, and a light chain CDR3 having an amino acid sequence of SEQ ID NO: 42; or
(c) a heavy chain variable region including a heavy chain CDR1 having an amino acid sequence of SEQ ID NO: 43, a heavy chain CDR2 having an amino acid sequence of SEQ ID NO: 44, and a heavy chain CDR3 having an amino acid sequence of SEQ ID NO: 45; and
a light chain variable region including a light chain CDR1 having an amino acid sequence of SEQ ID NO: 46, a light chain CDR2 having an amino acid sequence of SEQ ID NO: 47, and a light chain CDR3 having an amino acid sequence of SEQ ID NO: 48.

2. The anti-HLA-G CAR of claim 1, wherein the antigen-binding domain specifically binding to HLA-G is an antibody or an antigen-binding fragment.

3. The anti-HLA-G CAR of claim 2, wherein the antigen-binding fragment is a single-chain variable fragment (scFv).

4. The anti-HLA-G CAR of claim 1, wherein the CAR further comprises a leader sequence.

5. The anti-HLA-G CAR of claim 4, wherein the leader sequence is a leader sequence derived from cluster of differentiation 8 alpha (CD8α), a human immunoglobulin heavy chain, or a human granulocyte-macrophage colony-stimulating factor (hGM-CSF) receptor α-chain.

6. The anti-HLA-G CAR of claim 5, wherein the CD8α-derived leader sequence includes an amino acid sequence of SEQ ID NO: 8.

7. The anti-HLA-G CAR of claim 1, wherein the antigen-binding domain specifically binding to HLA-G is connected to the transmembrane domain by a hinge.

8. The anti-HLA-G CAR of claim 7, wherein the hinge is selected from the group consisting of a hinge of CD8α, a hinge of IgG1, a hinge of IgG4, a hinge of IgD, IgG1 CH3, an extracellular domain of CD28, or a combination thereof.

9. The anti-HLA-G CAR of claim 8, wherein the hinge of CD8α includes an amino acid sequence of SEQ ID NO: 10.

10. The anti-HLA-G CAR of claim 1, wherein the transmembrane domain is a transmembrane domain of a protein selected from the group consisting of CD8α, T cell receptor, CD28, CD3 epsilon (CD3ε), CD45, CD4, CD5, CD9, CD16, CD22, CD33, CD37, CD64, CD80, CD86, CD99, CD134, CD137, and alpha (α), beta (β), or zeta (ζ) of CD154.

11. The anti-HLA-G CAR of claim 10, wherein the transmembrane domain of CD8α includes an amino acid sequence of SEQ ID NO: 12.

12. The anti-HLA-G CAR of claim 1, wherein the intracellular signaling domain is derived from a protein selected from the group consisting of: CD3ζ, Fcy receptor (FcyR), inducible T-cell costimulatory (ICOS; CD278), 4-1BB (CD137, tumor necrosis factor receptor superfamily member 9 (TNFRSF9)), OX40 (CD134), CD27, CD28, interleukin-2 receptor beta (IL-2Rβ), IL-15R-α, CD40, myeloid differentiation primary response 88 (MyD88), DNAX-activating protein 10 (DAP10), DAP12, major histocompatibility complex (MHC) class I molecules, tumor necrosis factor (TNF) receptor proteins, immunoglobulin-like proteins, cytokine receptors, integrins, signaling lymphocytic activation molecule (SLAM) protein, activating NK cell receptors, B and T lymphocyte attenuator (BTLA), toll-like receptors (TLRs), CD2, CD7, CD30, CD99, CDS, intercellular adhesion molecule-1 (ICAM-1), B7-H3 (CD276), glucocorticoid-induced tumor necrosis factor receptor (TNFR)-related protein (GITR), B-cell activating factor (BAFF) receptor (BAFFR), lymphotoxin-β receptor interacting cytokine (LIGHT), herpesvirus entry mediator (HVEM) (LIGHT receptor (LIGHTR)), killer cell immunoglobulin like receptors, two Ig domains and short cytoplasmic tail 2 (KIRDS2), signaling lymphocytic activation molecule (SLAM) family member 7 (SLAMF7), NKp80 (killer cell lectin-like receptor subfamily F, member 1 (KLRF1)), NKp44, NKp30, NKp46, CD19, CD4, CD8α, CD8β, IL2Ry, IL7Rα, integrin subunit alpha 4 (ITGA4), very late antigen-1 (VLA1), CD49a, IA4, CD49D, integrin subunit alpha 6 (ITGA6), VLA-6, CD49f, integrin subunit alpha D (ITGAD), CD11d, integrin subunit alpha E (ITGAE), CD103, integrin subunit alpha L (ITGAL), CD11a, lymphocyte function-associated antigen-1 (LFA-1; CD11a/CD18), integrin subunit alpha M (ITGAM), CD11b, integrin subunit alpha X (ITGAX), CD11c, integrin beta-1 (ITGB1), CD29, integrin beta-2 (ITGB2), CD18, integrin beta-7 (ITGB7), natural killer group 2 member D (NKG2D; killer cell lectin like receptor K1 (KLRK1)), natural killer group 2 member C (NKG2C; (killer cell lectin like receptor C2 (KLRC2)), tumor necrosis factor receptor 2 (TNFR2), TNF-related activation-induced cytokine (TRANCE; receptor activator of nuclear factors κB ligand (RANKL)), DNAX accessory molecule-1 (DNAM1; CD226), SLAMF4 (CD244, 2B4), CD84, CD96 (tactile), carcinoembryonic antigen-related cell adhesion molecule 1 (CEACAM1), cytotoxic and regulatory T cell molecule (CRTAM), Ly9 (CD229), CD160 (BY55), P-selectin glycoprotein ligand 1 (PSGL1; CD162), CD100 (Semaphorin 4D (SEMA4D)), CD69, SLAMF6 (NK-T-B-antigen (NTB-A)), Ly108, SLAMF1 (CD150, IPO-3), B lymphocyte activation modulator and enhancer (BLAME; SLAMF8), lymphotoxin-beta receptor (LTBR), linker for activation of T cells (LAT), glutamate decarboxylase (GADS), Src sequence homology 2 (SH2) domain-containing leukocyte phosphoprotein of 76-kDa (SLP-76), phosphoprotein associated with glycosphingolipid-enriched microdomain/C-terminal Src kinase (Csk)-binding protein (PAG/Cbp), CD19a, and CD83-specific ligands.

13. The anti-HLA-G CAR of claim 12, wherein the CD3ζ-derived intracellular signaling domain includes an amino acid sequence of SEQ ID NO: 16.

14. The anti-HLA-G CAR of claim 1, wherein the intracellular signaling domain further includes a costimulatory domain derived from a protein selected from the group consisting of OX40, CD2, CD27, CD28, CD99, ICAM-1, LFA-1 (CD11a/CD18), ICOS (CD278), and 4-1BB (CD137).

15. The anti-HLA-G CAR of claim 14, wherein the 4-1BB-derived costimulatory domain includes an amino acid sequence of SEQ ID NO: 14.

16. An isolated nucleic acid comprising a nucleotide sequence encoding the anti-HLA-G CAR of any one of claims 1 to 15.

17. A vector comprising the isolated nucleic acid of claim 16.

18. An immune cell expressing the anti-HLA-G CAR of any one of claims 1 to 15 on a surface thereof.

19. The immune cell of claim 18, wherein the immune cell is selected from the group consisting of natural killer cells, T cells, natural killer T cells, B cells, macrophages, dendritic cells, natural killer dendritic cells, mast cells, and precursor cells thereof.

20. A pharmaceutical composition for use in the prevention or treatment of cancer, comprising the immune cell of claim 18 or claim 19 and a pharmaceutically acceptable carrier.

21. The pharmaceutical composition for use of claim 20, wherein the cancer is selected from the group consisting of pancreatic cancer, breast cancer, ovarian cancer, glioma, cervical cancer, endometrial cancer, esophageal cancer, stomach cancer, liver cancer, lung cancer, colon cancer, nasopharyngeal cancer, oral cancer, thyroid cancer, prostate cancer, renal cancer, gallbladder cancer, bile duct cancer, blood cancer, and melanoma.

22. A method for use in preventing or treating cancer, comprising administering the immune cell of claim 18 or claim 19, or the pharmaceutical composition of claim 20, to a subject in need thereof.
